# EUROPEAN PATENT APPLICATION

(11) **EP 4 316 513 A1**
(43) Date of publication of application: **07.02.2024**
(21) Application number: 22382749.4
(22) Date of filing: 02.08.2022
(51) Int. Cl.: A61K 39/12, A61P 31/14, C12N 15/85

(54) **NEW DNA SARS-COV-2 VACCINE**

(71) Applicant: Consejo Superior de Investigaciones Científicas (CSIC), 28006 Madrid (ES)
(72) Inventor: ALCOLEA ALCOLEA, Pedro José, 28040 Madrid (ES); LARRAGA RODRÍGUEZ DE VERA, Vicente, 28040 Madrid (ES); ALONSO AYALA, Ana María, 28040 Madrid (ES); LARRAGA CRIADO, Jaime, 28040 Madrid (ES); RUIZ GARCÍA, Silvia, 28040 Madrid (ES); LOAYZA, Francisco Javier, 28040 Madrid (ES); SEVILLA HIDALGO, Noemí, 28130 Madrid (ES)
(74) Representative: Hoffmann Eitle

(57) **Abstract**

The invention relates to combinations, pharmaceutical compositions and kits comprising polynucleotides encoding the spike (S) glycoprotein and the nucleocapsid (N) protein of SARS-CoV-2, either in a single or two separate vectors (e.g., a DNA plasmid), and their use in the prophylactic or therapeutic treatment of COVID-19.

## Description

### FIELD OF INVENTION

The invention relates to the field of vaccines, and generally relates to polynucleotide vaccines and vaccine compositions. More specifically, the invention relates to combinations, pharmaceutical compositions and kits comprising polynucleotides encoding the spike (S) glycoprotein and the nucleocapsid (N) protein of SARS-CoV-2, either in a single or two separate vectors (e.g., a DNA plasmid), and their use in the prophylactic or therapeutic treatment of COVID-19.

### BACKGROUND OF THE INVENTION

Despite the existence of several vaccines that protect from SARS-CoV-2 infection in developed countries, the COVID-19 pandemic is still a main concern worldwide due to the lack of vaccination in many countries and the emergence of new variants. These new viral strains of SARS-CoV-2, with several mutations in the S protein highlight the need for vaccines that provide a broader spectrum of protection that could cover future virus variant strains with modified S proteins.

Already, the presence of the Omicron variant (B.1.1.529), now dominant in many countries, which is not so sensitive to several of the usual vaccines, has emphasized the requirement for alternative vaccination strategies.

The proposed solution of additional vaccination doses for SARS-CoV-2 control does not seem to be useful to tackle the problem of vaccine escape variants.

DNA vaccines are one of the vaccine types to be considered in the future control of COVID-19 due to its advantages like their ability to be easily modified in short periods of time to adapt protection to new variants, as well as its thermotolerance, facilitating its distribution in low income countries, where the existence of the cold-chain is not always guaranteed. DNA vaccines could have a promising future in vaccinology since they are easy to manufacture and typically cheaper than other types [1].

In the context of the severe acute respiratory syndrome coronavirus 2 (SARS-CoV-2), the causative agent of the current COVID-19 pandemic, developing as many vaccines as possible is a priority.

Most vaccines currently undergoing development and those already available in the market are based solely on the SARS-CoV-2 spike (S) antigen [2], except for those based on a genetically modified SARS-CoV-2 virus.

In spite of the efforts made, however, there is still the need of further DNA-based vaccines with an efficient therapeutic effect in preventing or ameliorating COVID-19 disease in the population.

### SUMMARY OF THE INVENTION

The inventors have designed an efficient protective vaccine based on the combined administration of the spike (S) glycoprotein as well as the nucleocapsid (N) protein encoded nucleotide sequences of SARS-CoV-2.

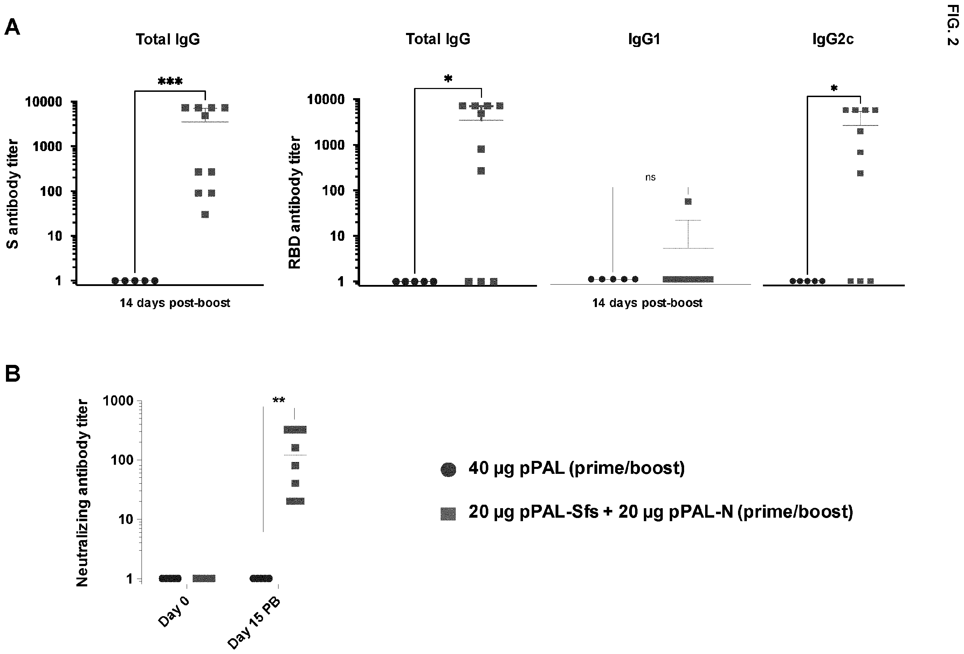

As it is shown below, when C57BL/6J mice were immunized with pPAL-Sfs + pPAL-N (20µg each), it was found that IgG2c/IgG1 ratio was higher than 100, indicating a balance toward Th1 response in pPAL-Sfs + pPAL-N vaccinated mice (Figure 2A). Due to the correlation of neutralizing antibodies (NAb) with protection, the SARS-CoV-2-specific NAb were assessed in sera samples from pPAL-Sfs + pPAL-N immunized mice. All animals showed significant neutralizing titers (>20, average equal to 100) after the booster dose (Figure 2B). Therefore, they concluded that the administration of a combination of pPAL-Sfs + pPAL-N polynucleotides elicited a SARS-CoV-2-specific humoral immune response in mice.

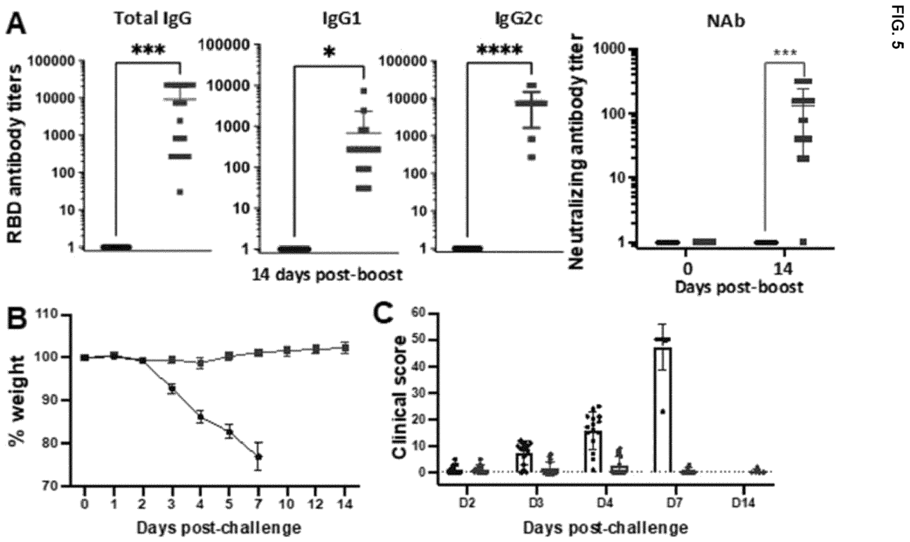

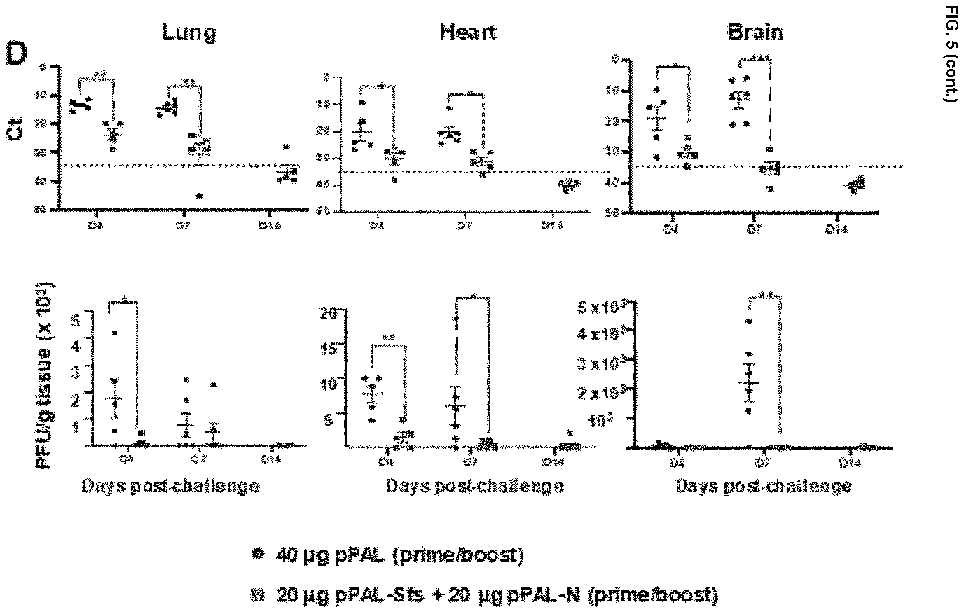

Not only that, but also, the inventors found that when transgenic mouse model K18-hACE2 were vaccinated with two doses of 20 µg of pPAL-Sfs + 20 µg of pPAL-N with a 15-day time interval, and 2 weeks later were challenged with a lethal dose of SARS-CoV-2, a complete protection against the disease was achieved in mice vaccinated with the two doses of the combination of plasmids containing Sfs and N encoding genes (Figure 5).

In order to show the synergistic effects of the combination, a comparative test was conducted between the combination of the invention comprising the administration of a polynucleotide encoding the SARS-CoV-2 S polypeptide (A) and a polypeptide encoding the SARS-CoV-2 N polypeptide (B) vs the administration of a polynucleotide encoding the SARS-CoV-2 S polypeptide (A) only. A naked plasmid was used as control.

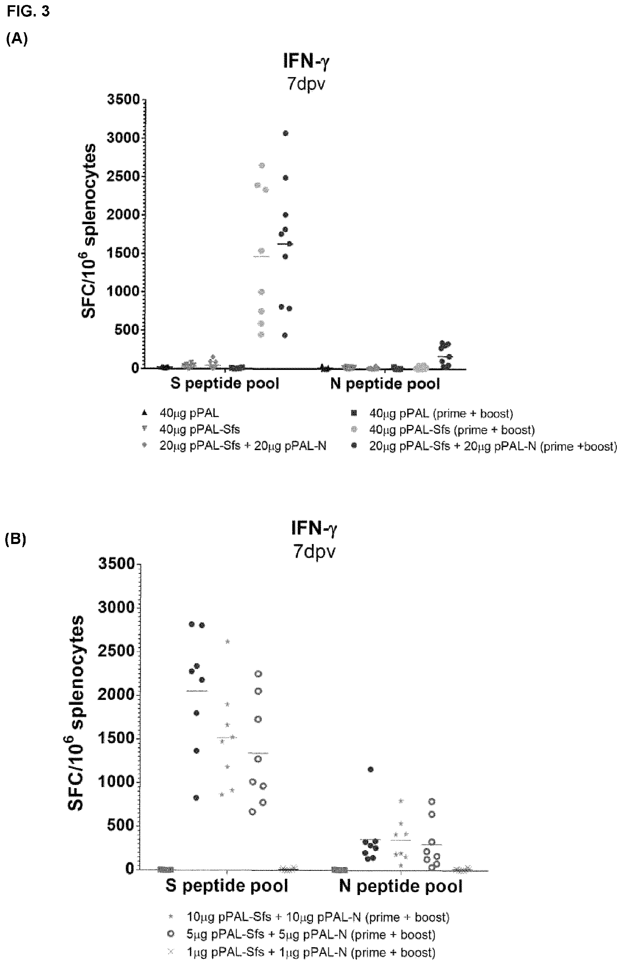

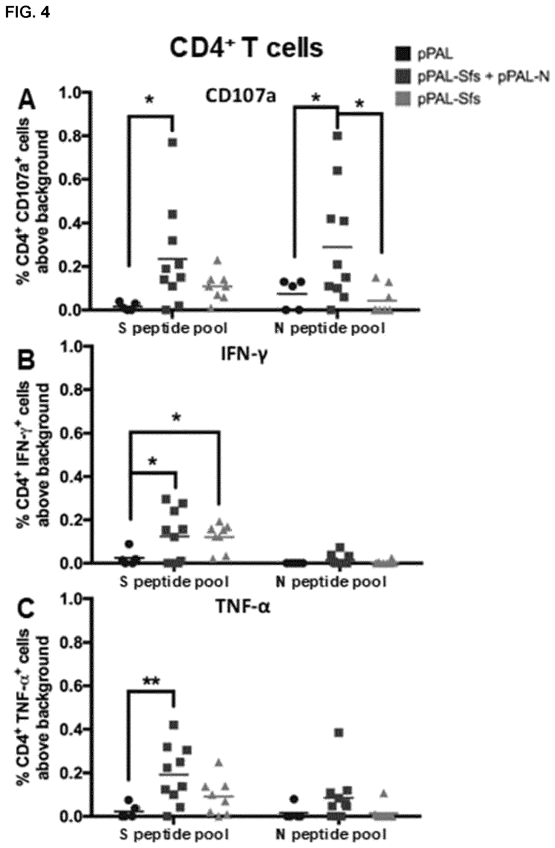

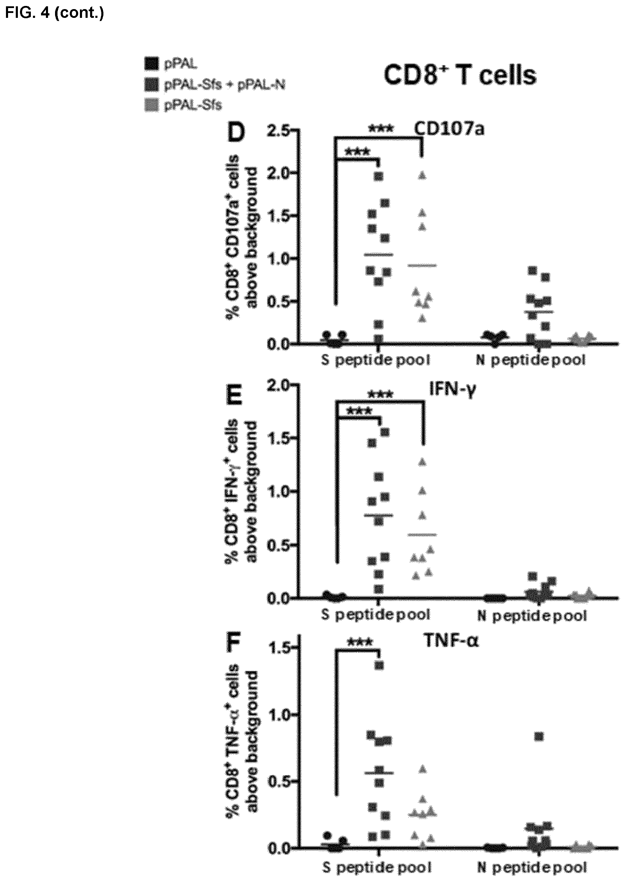

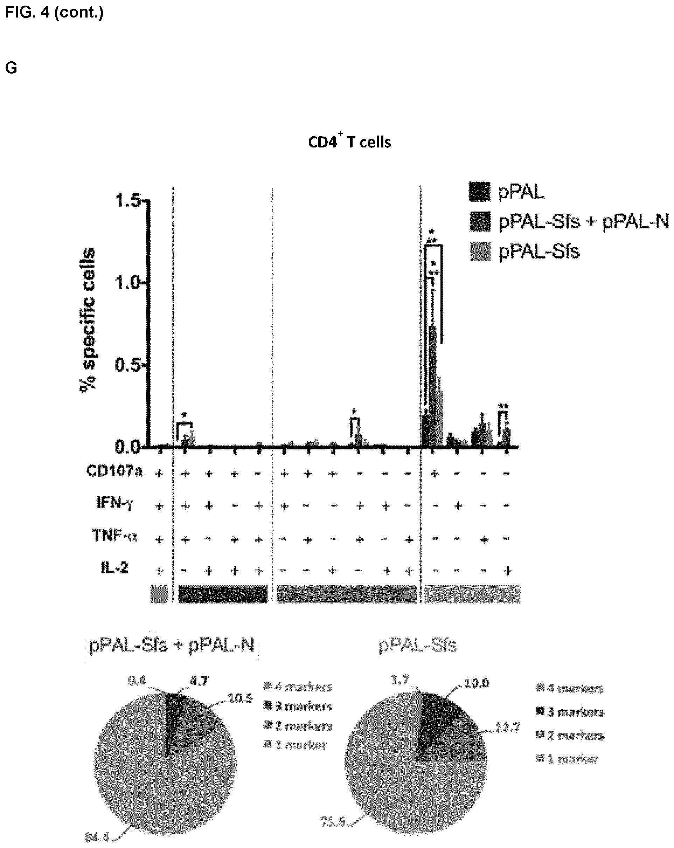

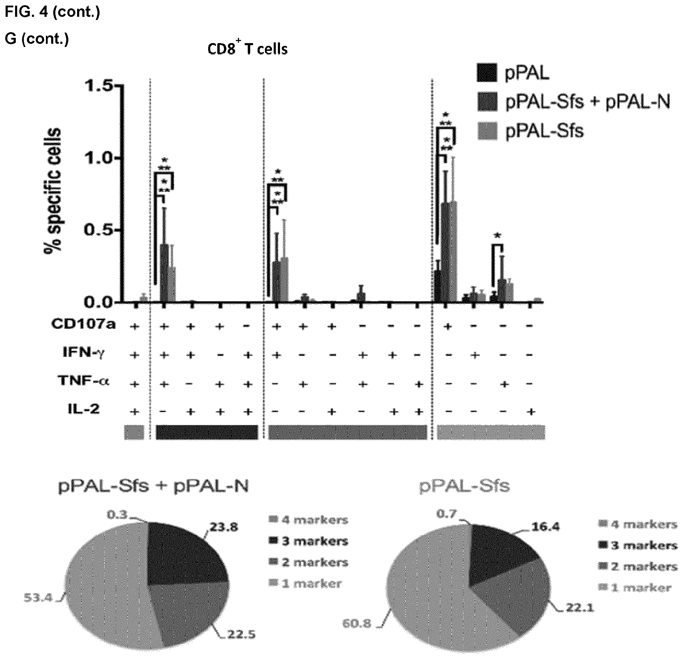

A very clear potentiating effect was observed in the cellular response when the pPAL-N and pPAL-Sfs plasmids were administered in a prime-boost regimen with respect to the different parameters analysed (Figs. 3 and 4). It shall be considered that for the single and combination regimens the total amount of inoculated DNA is the same (40 micrograms). Thus, the subgroup vaccinated only with pPAL-SfS received a 2x concentration, and still the combined treatment enabled achieving an improved response.

Moreover, viral load was drastically reduced in lungs, brain and heart of vaccinated animals (Fig. 5D). These data indicate that vaccinated animals are not only able to control the viral disease, but also to limit viral replication. Therefore, pPAL-Sfs + pPAL-N is a promising DNA vaccine candidate for protection of the murine animal model against the disease.

In view of the above, the combination of proteins S and N is capable to induce an efficient and long-term immunological memory.

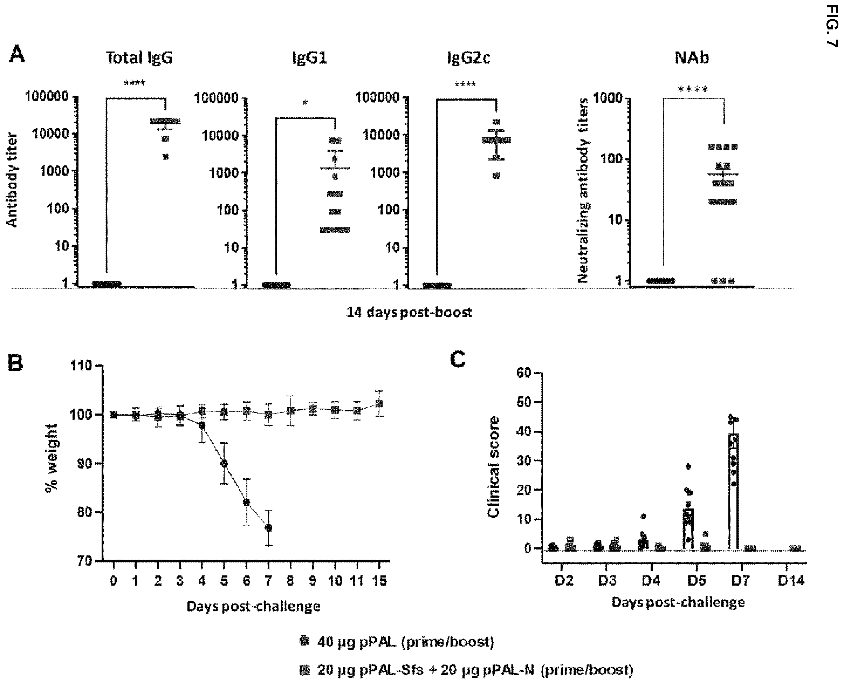

In addition to the above, the inventors found that the combination of S and N proteins induced protective effect against lethal challenge with the dominant B1.617.2 (Delta) variant, figures 7A-C.

Altogether, the present invention means a great advance in managing SARS-CoV-2, providing a combination which is able to elicit robust SARS-CoV-2-specific humoral and cellular immune responses, protecting mice from lethal challenge with the Wuhan isolate as well as the Delta variant.

Thus, in a first aspect of the invention relates to a combination comprising:
- a polynucleotide sequence A comprising or consisting of a nucleic acid sequence encoding a SARS-CoV-2 Spike (S) glycoprotein or an immunogenic fragment thereof; and
- a polynucleotide sequence B comprising or consisting of a nucleic acid sequence encoding a SARS-CoV-2 nucleocapsid (N) protein or an immunogenic fragment thereof.

The polynucleotides of said combination can be part of the same or separate expression vector or construct. Also, the polynucleotides or vectors of said combination can be part of the same or separate (pharmaceutical) compositions.

In one embodiment of the invention, optionally in combination with any of the embodiments provided above or below, each one of the polynucleotide sequences (A) and (B) forms part of a separate expression vector or construct. In an alternative embodiment, both polynucleotides (A) and (B) form part of the same expression vector or construct.

A second aspect of the invention relates to a pharmaceutical composition comprising a therapeutically effective amount of the combination as defined in the first aspect of the invention, further comprising one or more pharmaceutically acceptable carrier(s), additive(s) or excipient(s).

A third aspect of the invention relates to a kit of parts comprising:
- the polynucleotide sequence A as defined in the first aspect of the invention in a first unit dosage form,
- the polynucleotide sequence B as defined in the first aspect of the invention in a second unit dosage form,
- container means for containing said first and second dosage forms;
- and optionally, instructions for use.

A fourth aspect of the invention relates to a combination as defined in the first aspect of the invention for use as a medicine, particularly as a vaccine.

A fifth aspect of the invention relates to a combination, pharmaceutical composition or a kit as defined in any of the above aspects for use in a method for preventing or treating COVID-19 and/or persistent COVID-19 in a subject, the method comprising the simultaneous, separate or sequential administration of the polynucleotides A and B. This aspect can be formulated as the use of a combination or pharmaceutical composition as defined in any of the above aspects for the manufacture of a medicament for preventing or treating COVID-19 and/or persistent COVID-19 in a subject, by the simultaneous, separate or sequential administration of the polynucleotides A and B. This aspect can be alternatively formulated as a method for preventing or treating COVID-19 and/or persistent COVID-19 in a subject, the method comprising the simultaneous, separate or sequential administration of the polynucleotides A and B in a therapeutically effective amount to the subject in need thereof.

A sixth aspect of the invention relates to a method for the obtaining of antibodies comprising the immunization of a mammal other than a human, such as a non-human primate, a cow, a goat, a pig or a horse, with the combination, the pharmaceutical composition or the kit as defined in any of the preceding aspects.

A seventh aspect of the invention relates to an antibody or an antigen-binding fragment thereof, obtained or obtainable after immunization of a mammal with the combination, pharmaceutical composition or kit as defined in any of the above aspects, wherein said antibody or fragment thereof is suitable for the prophylactic or therapeutic treatment of a mammalian subject.

In an eighth aspect the invention relates to a polynucleotide sequence SEQ ID NO: 6.

In a final aspect the invention relates to a polypeptide sequence encoded by SEQ ID NO: 6.

### BRIEF DESCRIPTION OF THE DRAWINGS

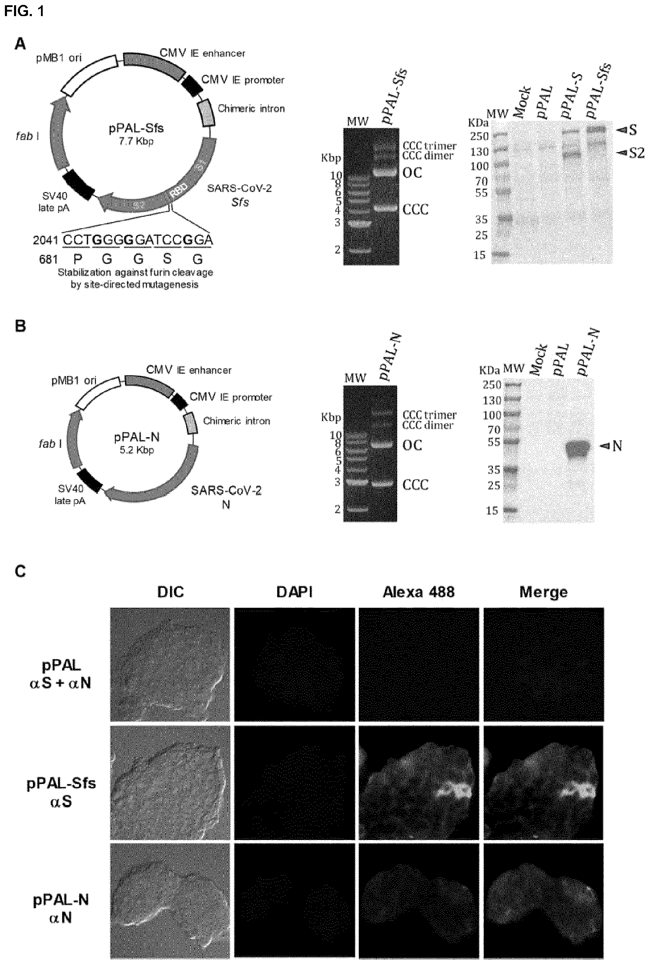

**Figure 1****.** The non-replicative antibiotic resistance gene-free vaccinal pPAL-Sfs (SEQ ID NO: 11) and pPAL-N (SEQ ID NO: 13) constructs. **(A)** pPAL-Sfs map, including Sfs resistant to furin cleavage and topological conformers visualized by 1% agarose gel electrophoresis. Western blot of whole protein extracts from HEK293 cells transfected with pPAL, pPAL-S (SEQ ID NO: 9), and pPAL-Sfs (SEQ ID NO: 11). The primary antibody was 1:800-diluted goat anti-S polyclonal antibody (Abcam), which recognizes only the S2 subunit. **(B)** pPAL-N plasmid map and 1% agarose gel electrophoresis. Western blot of whole protein extracts from HEK293 cells transfected with pPAL, and pPAL-N showing N expression in HEK293 transfected cells. The primary antibody was 1:500-diluted rabbit anti-N polyclonal antibody (C). Immunofluorescence of cultured HEK293 transfected with pPAL, pPAL-Sfs and pPAL-N plasmids
**Figure 2****.** The pPAL-Sfs + pPAL-N vaccine candidates activate SARS-CoV-2 S- and RBD-specific humoral immunity in C57BL/6J mice. The prime/boost regime was tested. The serum samples were collected 15 days after the booster dose (15dpv). **(A)** Individual titers and mean of total circulating anti-S and anti-RBD (receptor-binding domain) IgG determined by ELISA using HRP-conjugated protein A. Individual titers and mean of circulating anti-RBD IgG1, IgG2c determined using HRP-conjugated polyclonal goat anti-mouse IgG1, IgG2a, and IgG2b. **(B)** Neutralizing antibodies determined by plaque assay in VERO cell cultures.
**Figure 3****.** SARS-CoV-2 S- and N-specific cellular immune response activation (IFN-y production) in C57BL/6J mouse spleen after immunization with the pPAL-Sfs and the pPAL-Sfs + pPAL-N vaccine candidates. Splenocytes were obtained 7dpv from mice after single dose and prime/boost inoculation with pPAL (control group), pPAL-Sfs, and pPAL-Sfs + pPAL-N (vaccinated groups). Splenocytes were set onto IFN-γ ELIspot plates (2×10⁵ cells/well) and stimulated for 16 h with 11-mer peptide pools (25 µg peptides/well) representing the whole S and N amino acid sequences in equimolar amounts. The overlap between contiguous peptides was 8 amino acids. Negative stimulation controls were substracted for each individual sample. **(A)** Individual values and arithmetic mean of IFN-γ spot-forming colonies (SFC) per million cells are plotted. A strong cell immune response is observed only with the prime/boost regime of pPAL-Sfs + pPAL-N. The response is significantly higher against the S than against the N protein. A non-statistically significant enhancement of the S response by the presence of pPAL-N is observed, although the final DNA amount is the same in the pPAL-Sfs dose and in the pPAL-Sfs + pPAL-N mix dose. **(B)** pPAL-Sfs + pPAL-N dose-response correlation.- Individual values and arithmetic mean of IFN-γ spot-forming colonies (SFC) per million cells are plotted. The cell immune response decays with the pPAL-Sfs + pPAL-N dose.
**Figure 4****.** Specific CD3+CD4+ and CD3+CD8+ T cell response induced by pPAL-Sfs + pPAL-N and pPAL-Sfs in C57BL/6J mice. Sinergistic effect of the S and N antigens. Polyfunctionality analysis. Splenocytes were obtained 7dpv from mice after single dose and prime/boost inoculation with pPAL (control group), pPAL-Sfs, and pPAL-Sfs + pPAL-N (vaccinated groups). Splenocytes were stimulated for 4 h with 11-mer peptide pools (25 µg peptides/well) representing the whole S and N amino acid sequences in equimolar amounts. Representative dot plots selected in the T CD3+CD4+ cell region accounting for the percentage of (A) CD107a+, **(B)** IFN-γ+, and (C) TNF-α+. T CD3+CD8+ specific stimulation. Representative dot plots selected in the T CD3+CD8+ cell region accounting for **(D)** CD107a+, **(E)** IFN-γ+, and **(F)** TNF-a+cells. **(G)** T CD3+ cells polyfunctionality analysis. Statistical inference was performed using two-way ANOVA and the post-hoc LSD Fisher's test (** p<0.01; *** p<0.001).
**Figure 5****.** The pPAL-Sfs + pPAL-N vaccine confers full protection against 10⁵ pfu SARS-CoV-2 (Wuhan-Hu-1 strain, MAD6 isolate) challenge in the K18-hACE2 murine model. **(A)** ELISA titration of circulating anti-RBD IgG 15dpv and neutralizing antibodies. **(B)** Body weight evolution after 105 pfu SARS-CoV-2 challenge. **(C)** Clinical sign follow-up after 10⁵ pfu SARS-CoV-2 challenge. (**D**) ΔCt accounting for SARS-CoV-2 mRNA levels. β-actin was the reference gene and viral replication assay from target organs (lungs, heart, and brain) tissue samples.

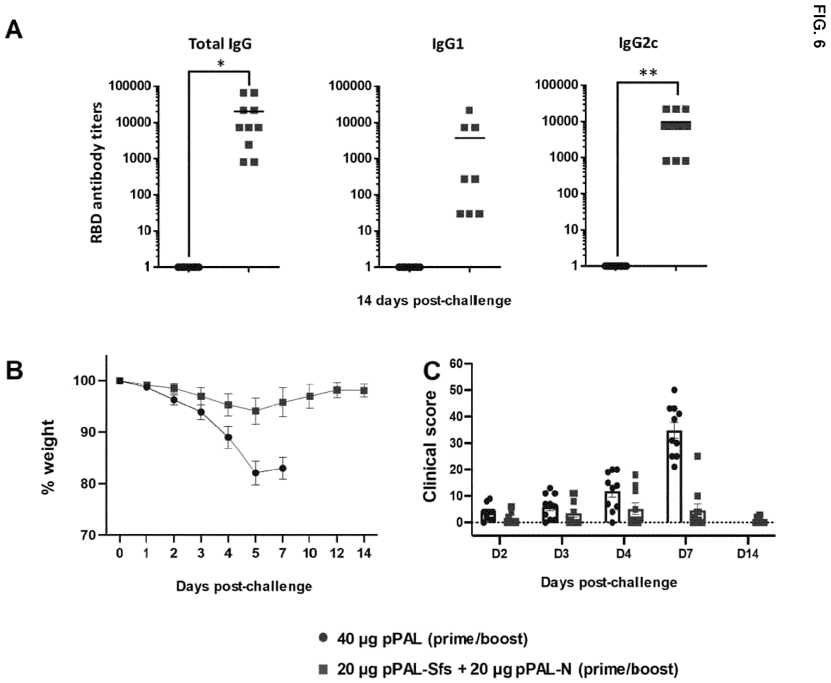

**Figure 6****.** The pPAL-Sfs+ pPAL-N vaccine confers full protection against 10⁵ pfus SARS-CoV-2 (Wuhan-Hu-1 strain, MAD6 isolate) challenge in the K18-hACE2 murine model after three months of vaccination. **(A)** Individual titers and mean of circulating anti-RBD specific IgG, IgG1, and IgG2c antibodies. **(B)** Body weight evolution after 10⁵ pfu SARS-CoV-2 challenge. **(C)** Clinical sign follow-up after 10⁵ pfu SARS-CoV-2 challenge. Statistical inference was performed using two-way ANOVA and post-hoc LSD Fisher's test (** p<0.01; *** p<0.001) indicate statistical significance in the differences between vaccinated (red points) and non-vaccinated (blue points) animals.
**Figure 7****.** The pPAL-Sfs+ pPAL-N vaccines confers full protection against 10⁵ pfus SARS-CoV-2 (Delta variant B1.617.2) challenge in the K18-hACE2 murine model after three months of vaccination. **(A)** Individual titers and mean of circulating anti-RBD specific IgG, IgG1, and IgG2c antibodies, and neutralizing antibodies. **(B)** Body weight evolution after 10⁵ pfu SARS-CoV-2 challenge. **(C)** Clinical sign follow-up after 10⁵ pfu SARS-CoV-2 challenge. Statistical inference was performed using two-way ANOVA and post-hoc LSD Fisher's test (** p<0.01; *** p<0.001) indicate statistical significance in the differences between vaccinated (red points) and non-vaccinated (blue points) animals.

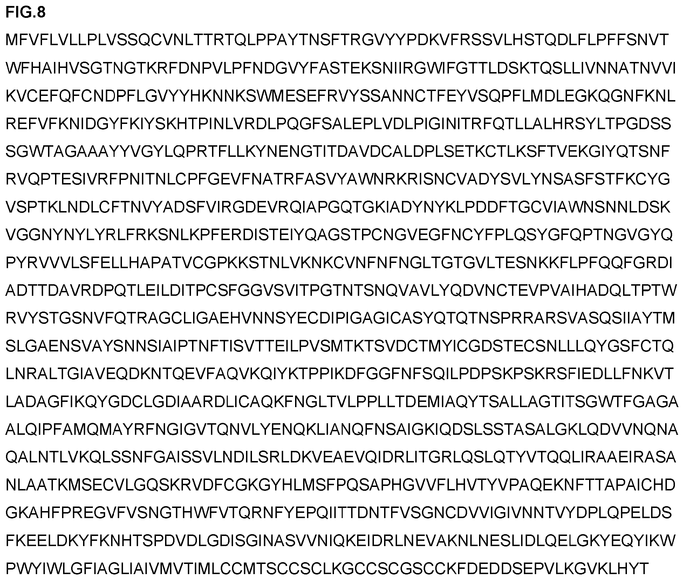

**Figure 8****.** It corresponds to SEQ ID NO: 1; the protein S amino acid sequence (Wuhan-Hu-1 strain). NCBI NC045512.2. Protein ID YP_009724390.1.

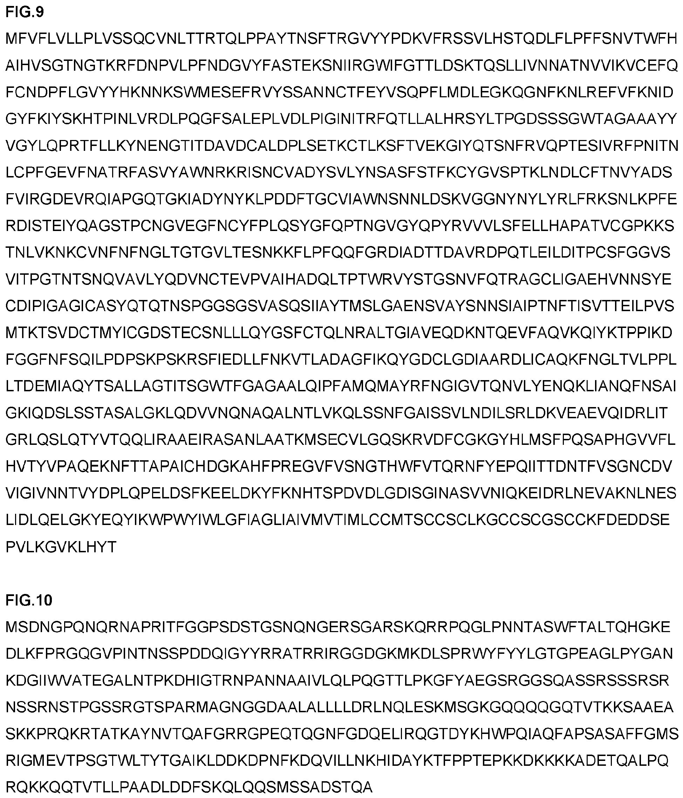

**Figure 9****.** It corresponds to SEQ ID NO: 2; the Sfs amino acid sequence, wherein the modified sequence to prevent furin cleavage is underlined.
**Figure 10****.** It corresponds to SEQ ID NO: 3; the protein N amino acid sequence (Wuhan-Hu-1 strain). NCBI NC045512.2. Protein ID YP_009724397.2.

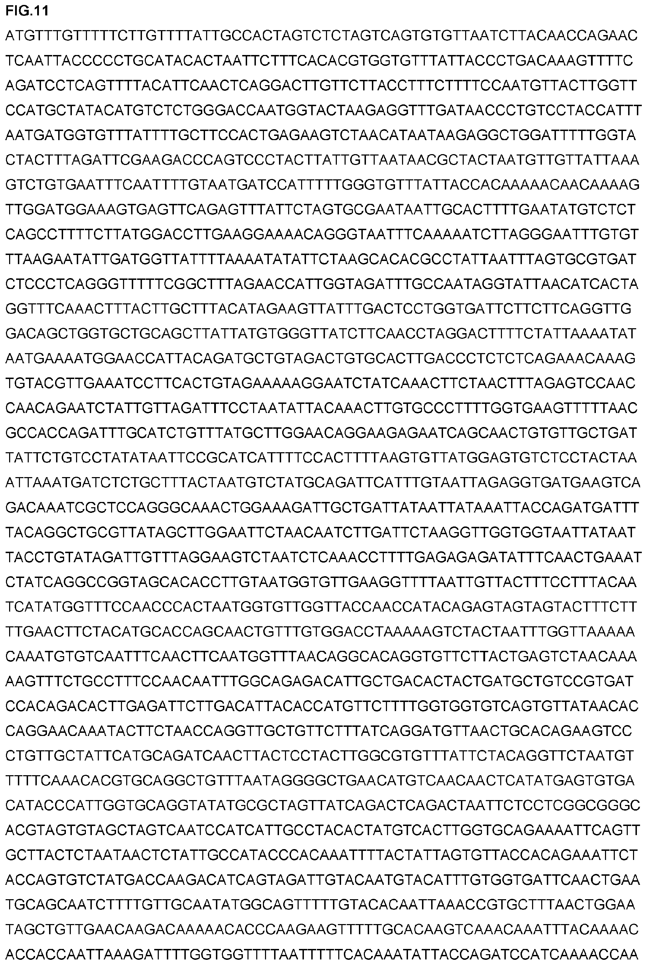

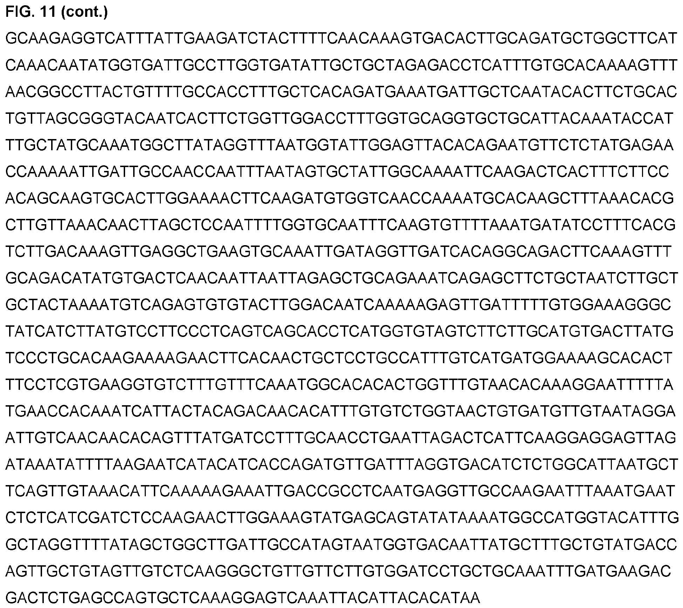

**Figure 11****.** It corresponds to SEQ ID NO: 4; the S nucleotide sequence (Wuhan-Hu-1 strain). NCBI NC045512.2. Gene ID: 43740568). 3822 bp.

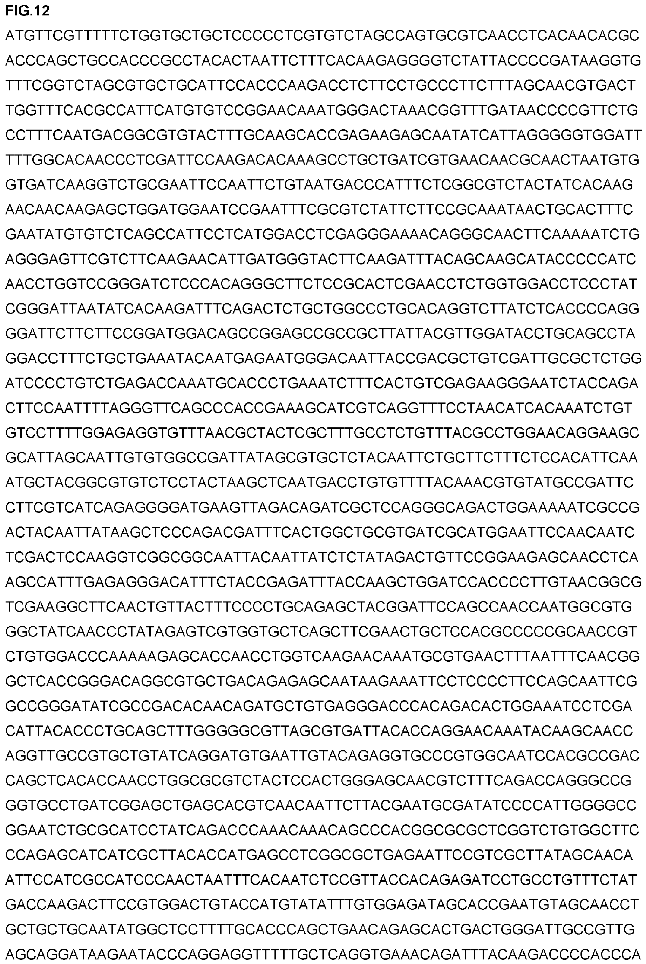

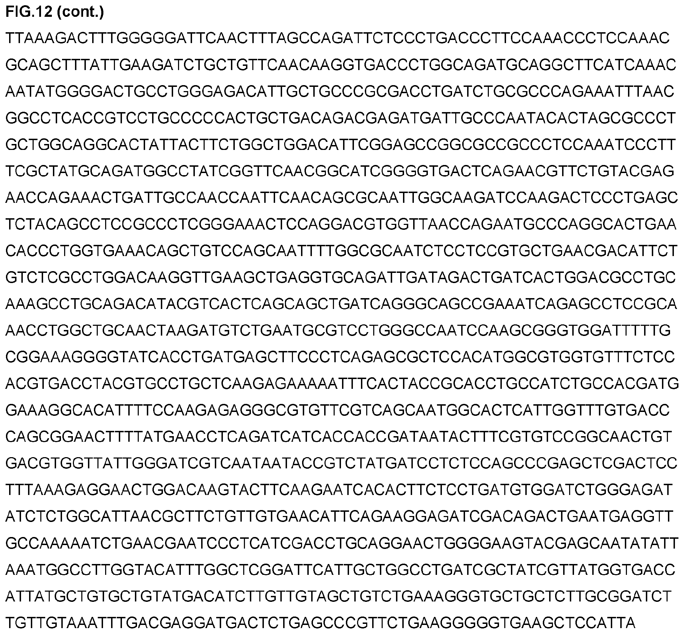

**Figure 12****.** It corresponds to SEQ ID NO: 5; the codon-optimized for expression in human S nucleotide sequence.

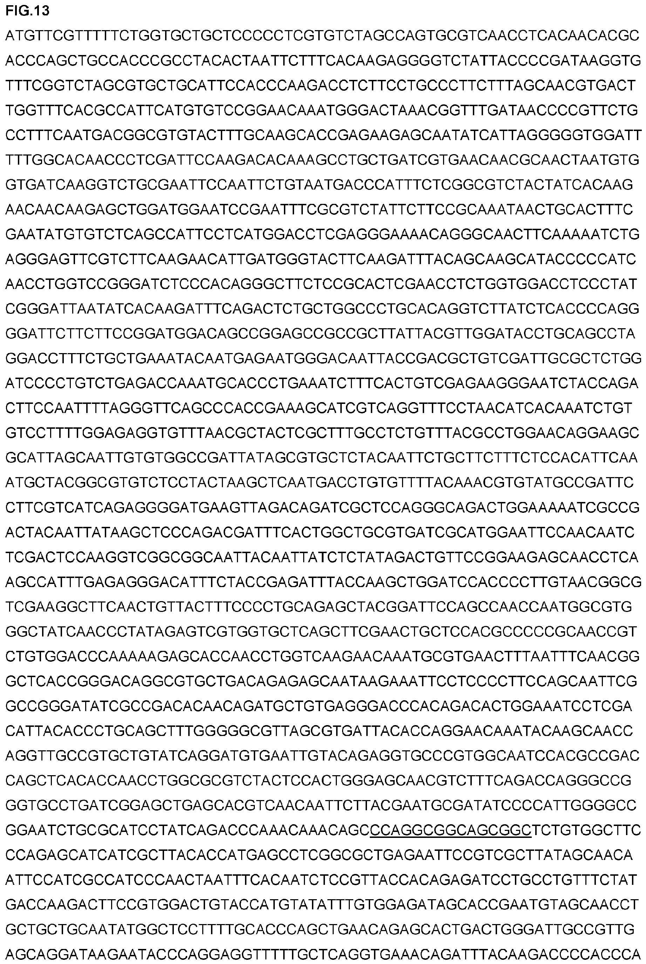

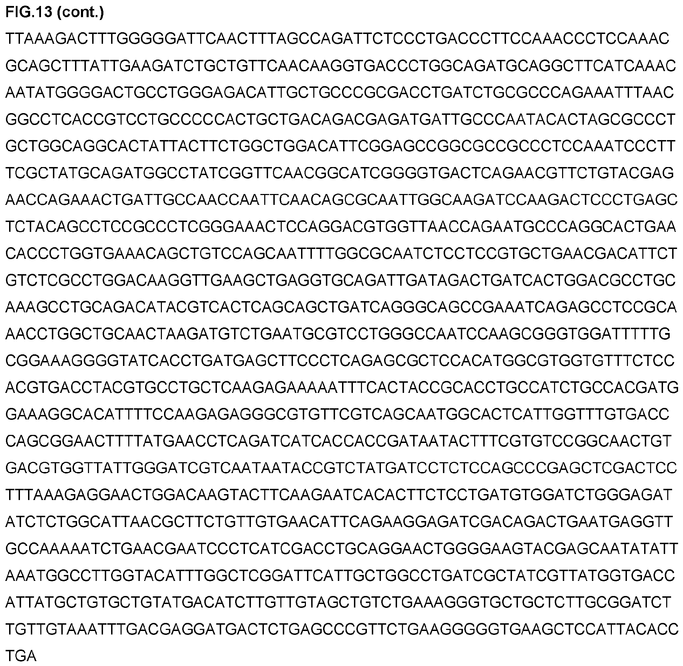

**Figure 13****.** It corresponds to SEQ ID NO: 6; the codon-optimized for expression in human S nucleotide sequence with a modification in the sequence encoding the furin cleavage site (Sfs) - underlined.

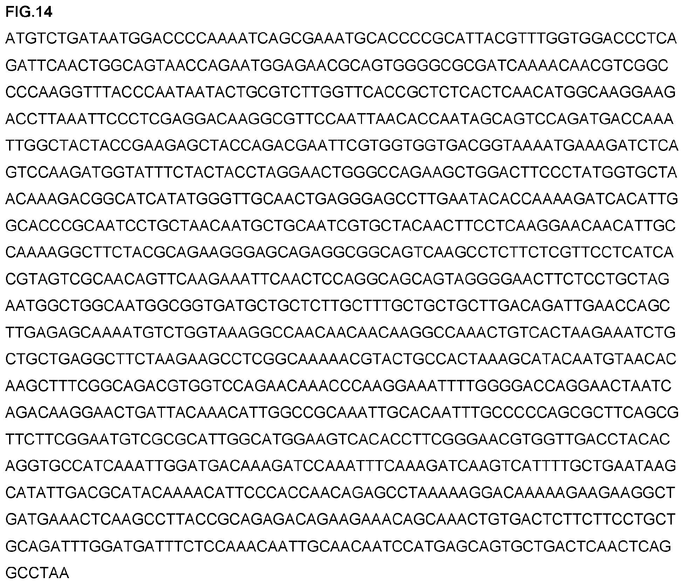

**Figure 14****.** It corresponds to SEQ ID NO: 7; the N nucleotide sequence (Wuhan-Hu-1 strain). NCBI NC045512.2. Gene ID: 43740575.

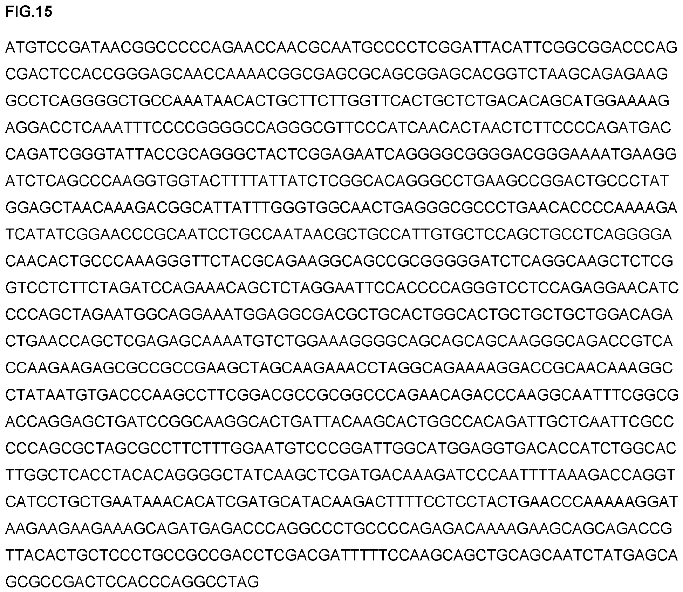

**Figure 15****.** It corresponds to SEQ ID NO: 8; the codon-optimized for expression in human N nucleotide sequence.

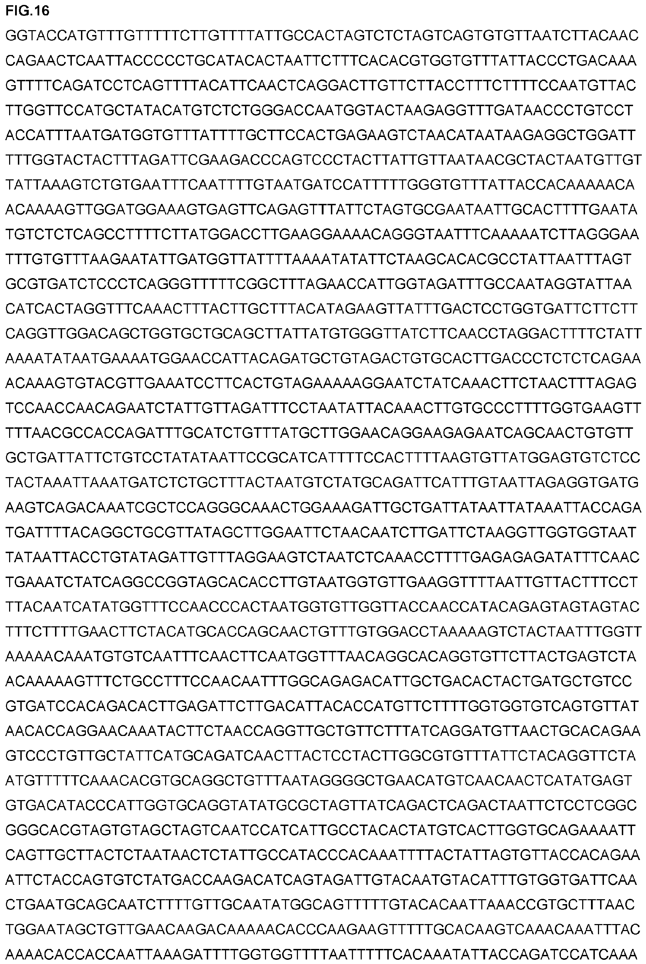

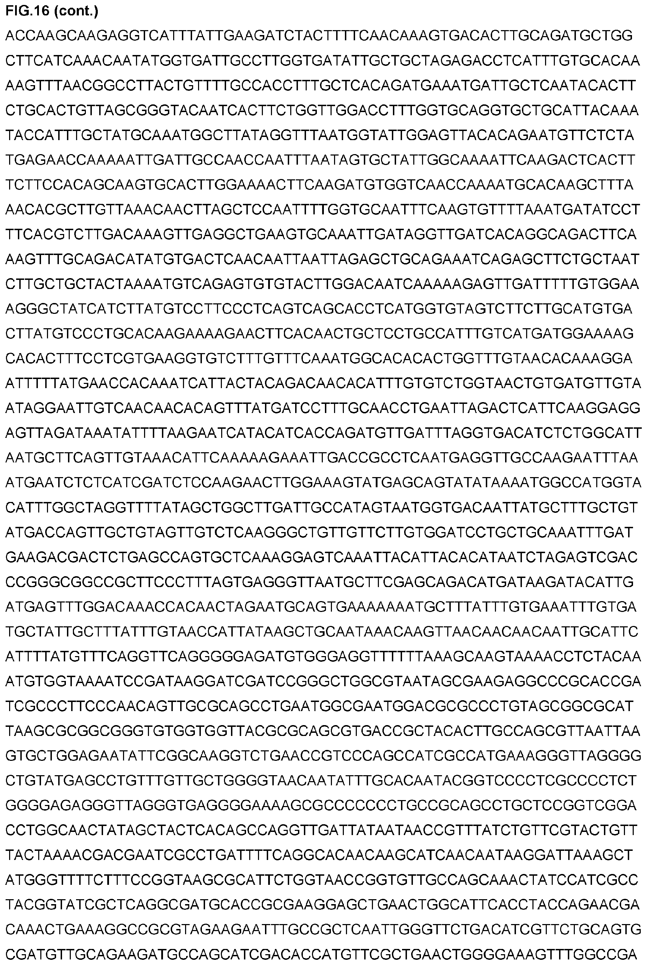

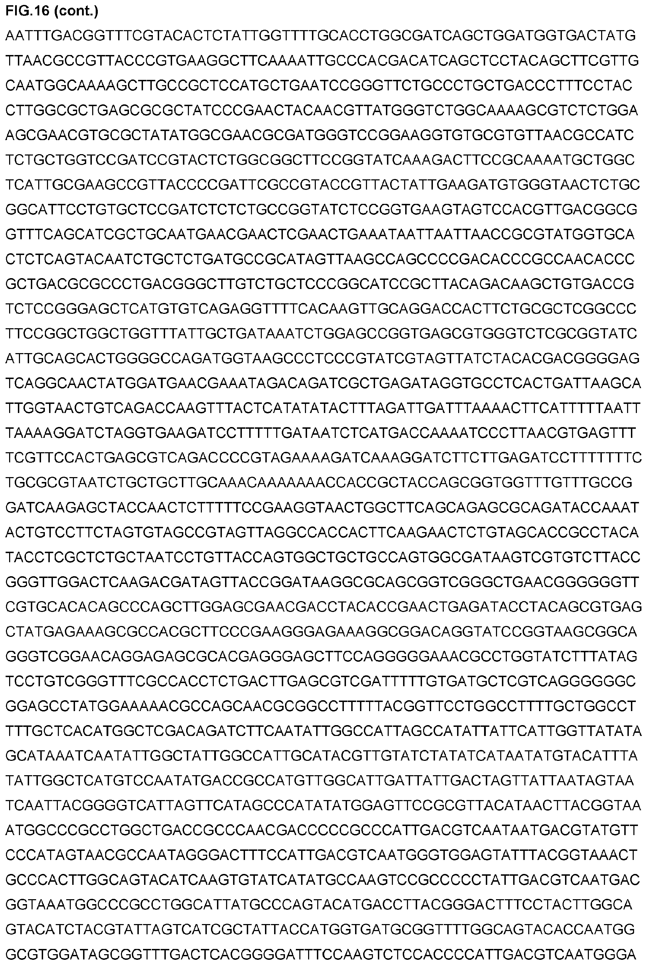

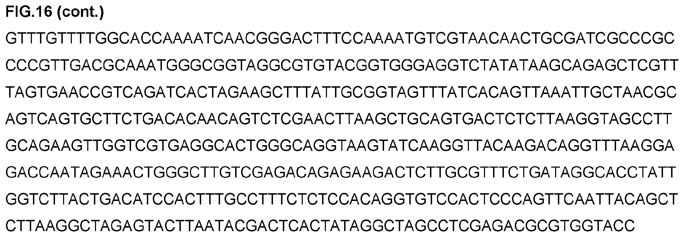

**Figure 16****.** It corresponds to SEQ ID NO: 9; the pPAL-S.

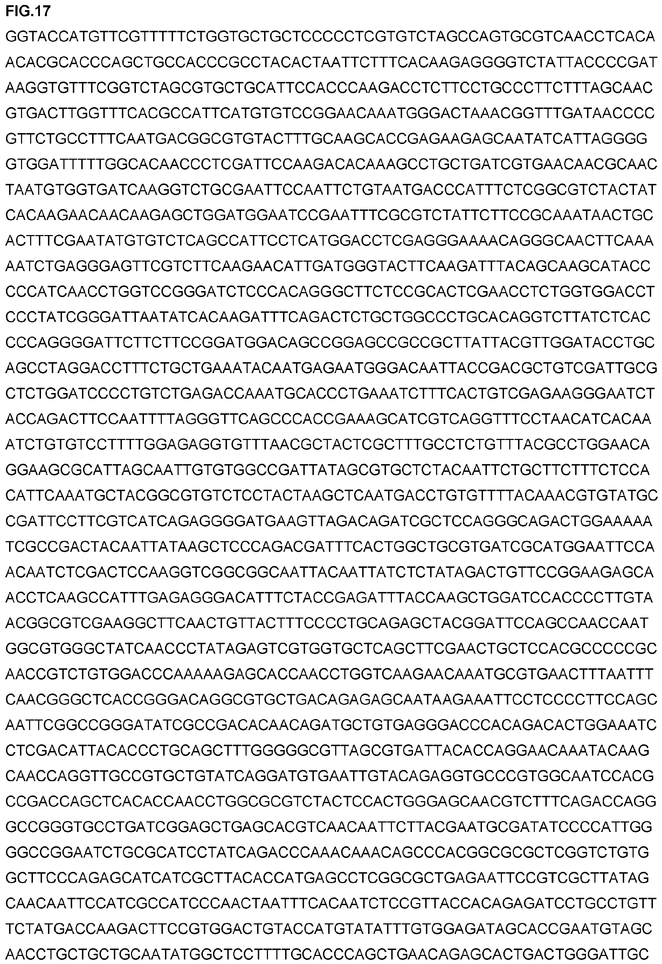

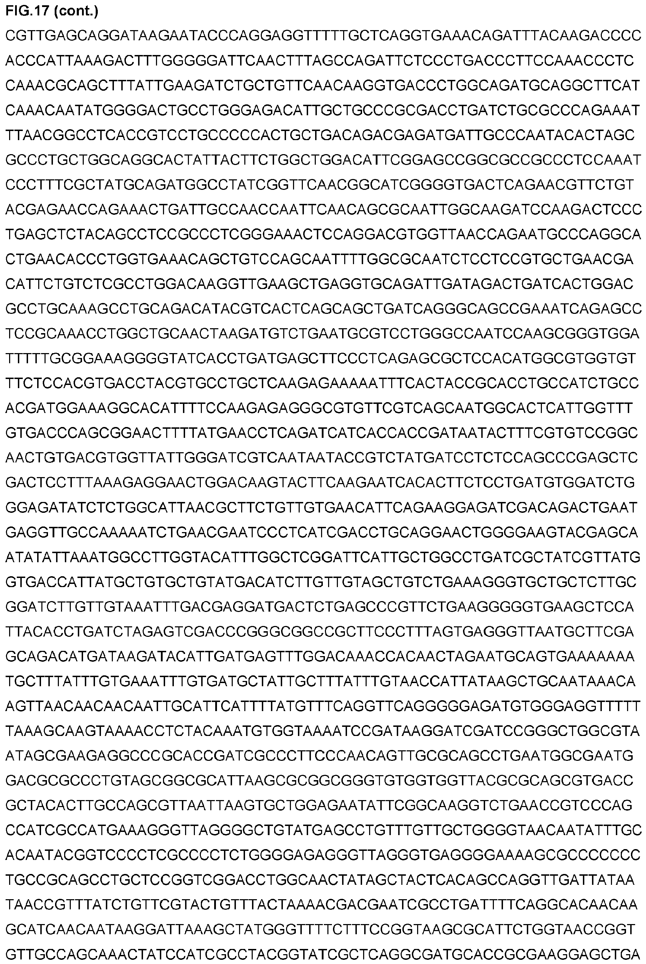

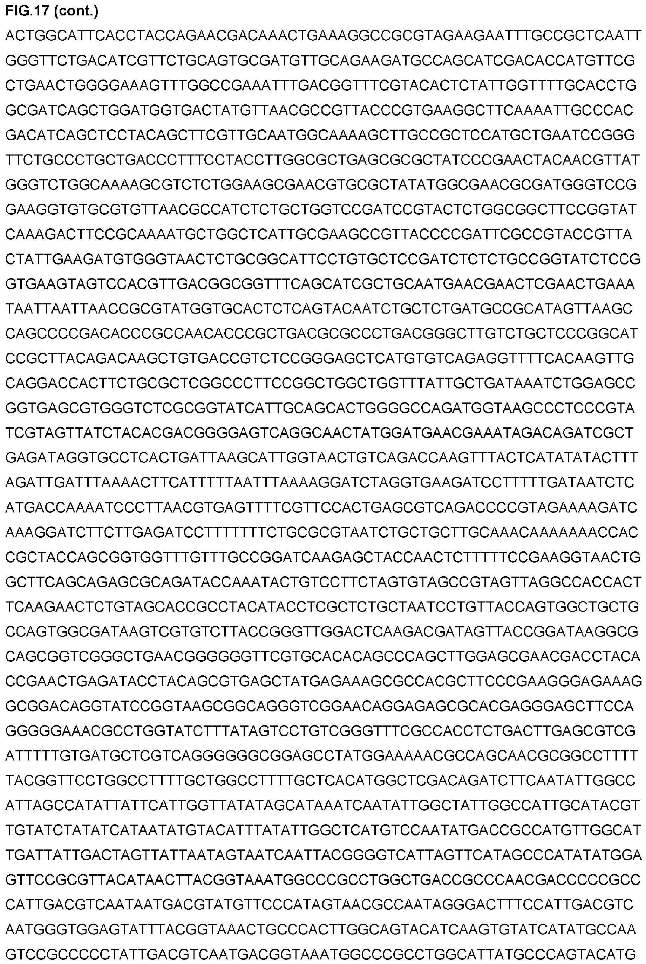

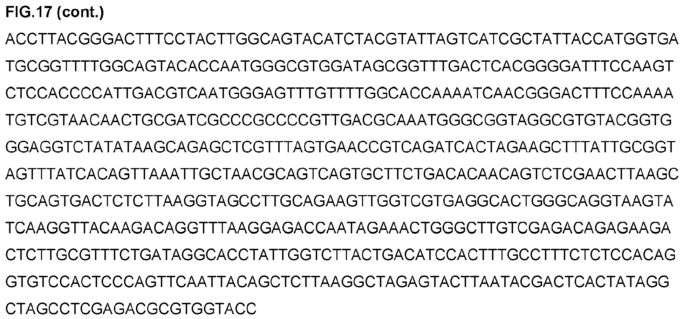

**Figure 17****.** It corresponds to SEQ ID NO: 10; the pPAL-S (codon-optimized S gene).

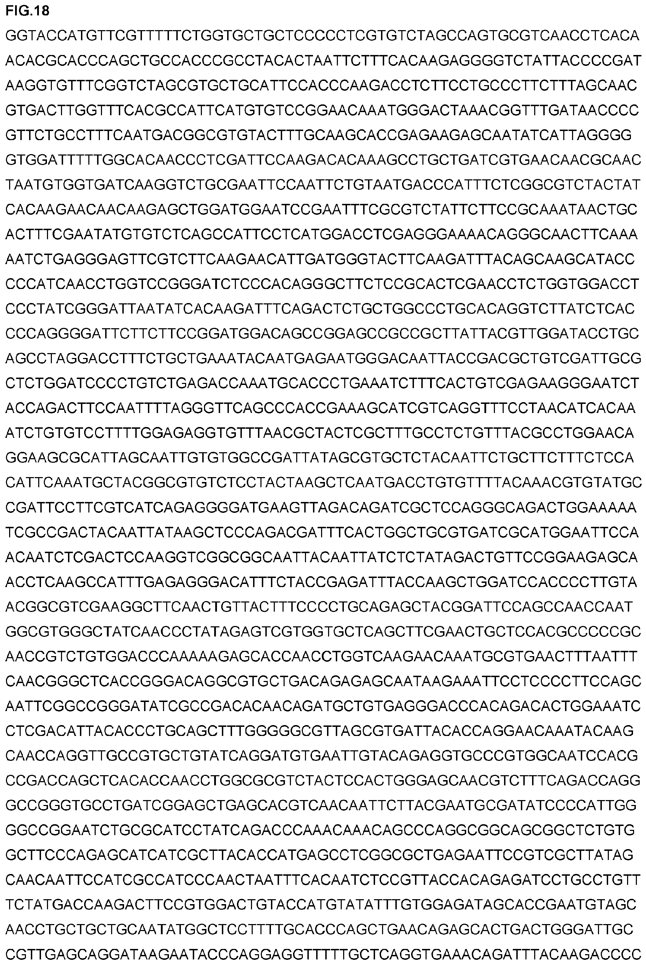

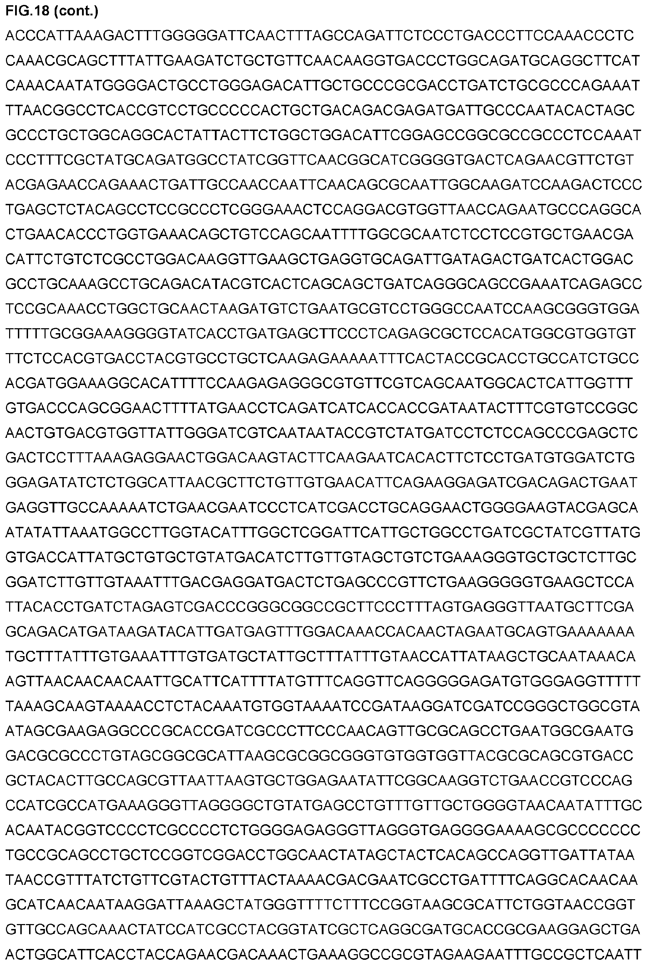

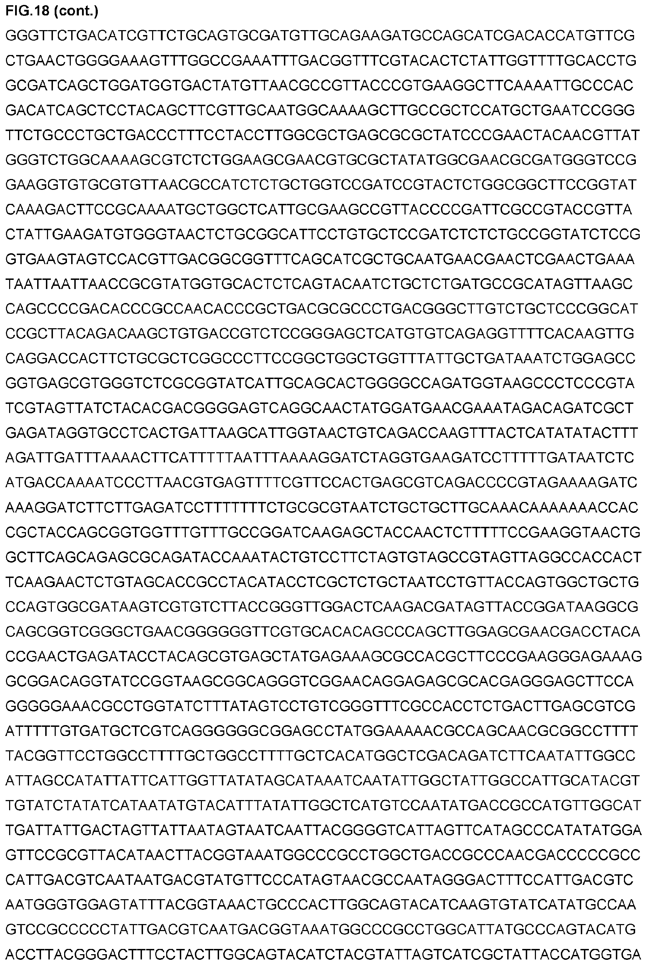

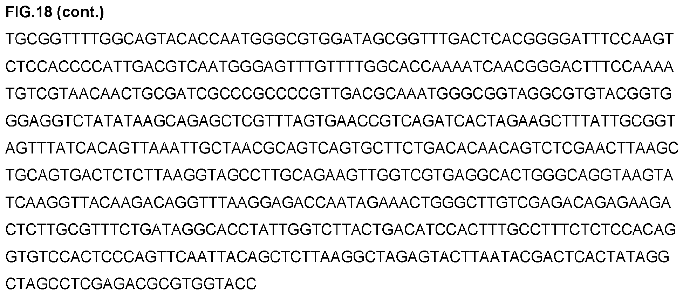

**Figure 18****.** It corresponds to SEQ ID NO: 11; the pPAL-Sfs (codon-optimized Sfs gene).

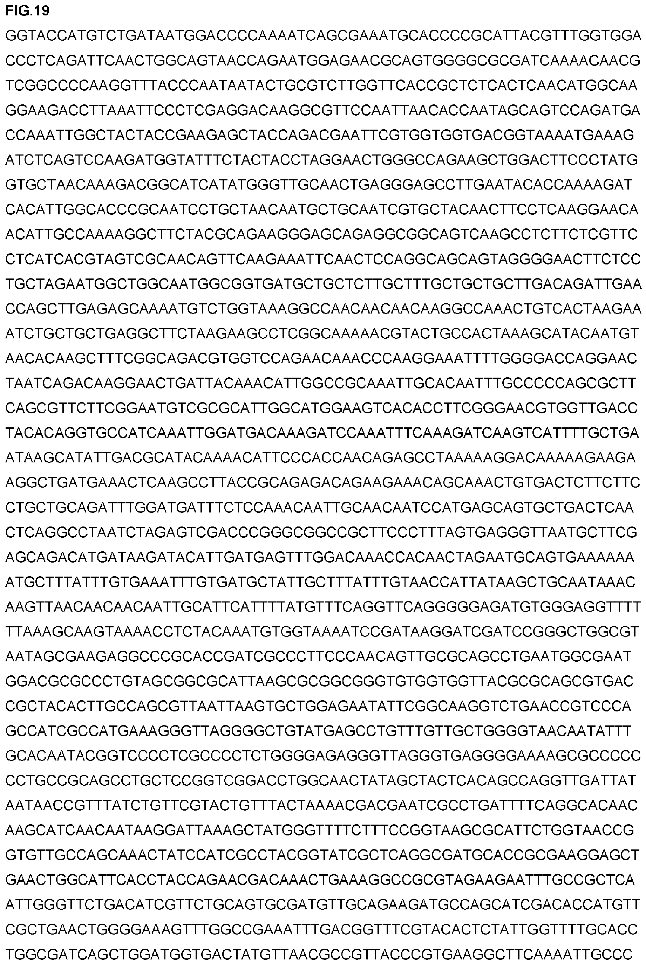

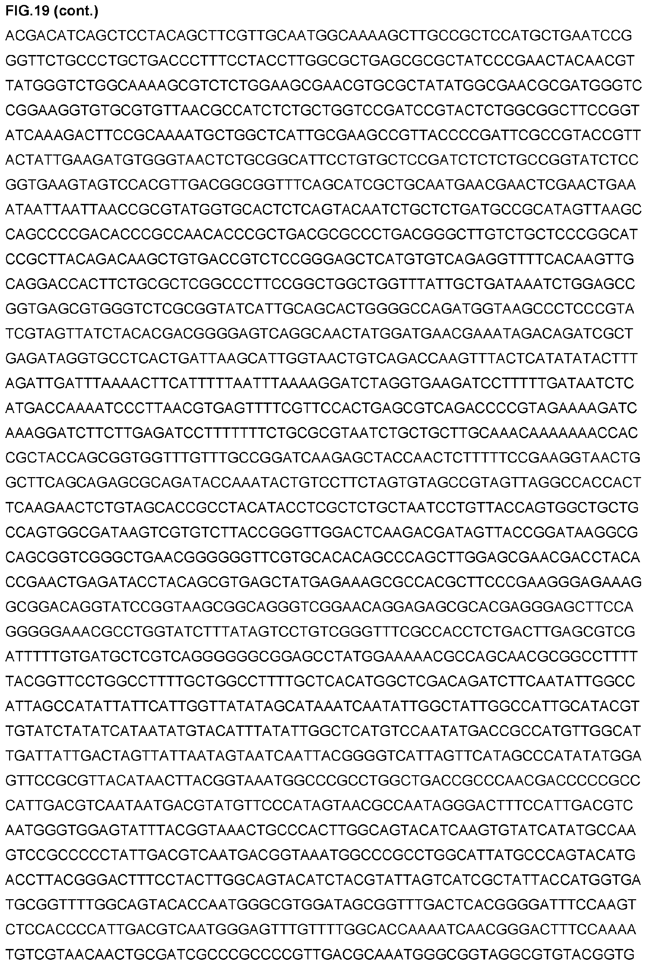

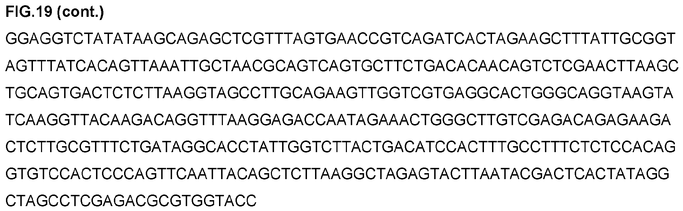

**Figure 19****.** It corresponds to SEQ ID NO: 12; the pPAL-N.

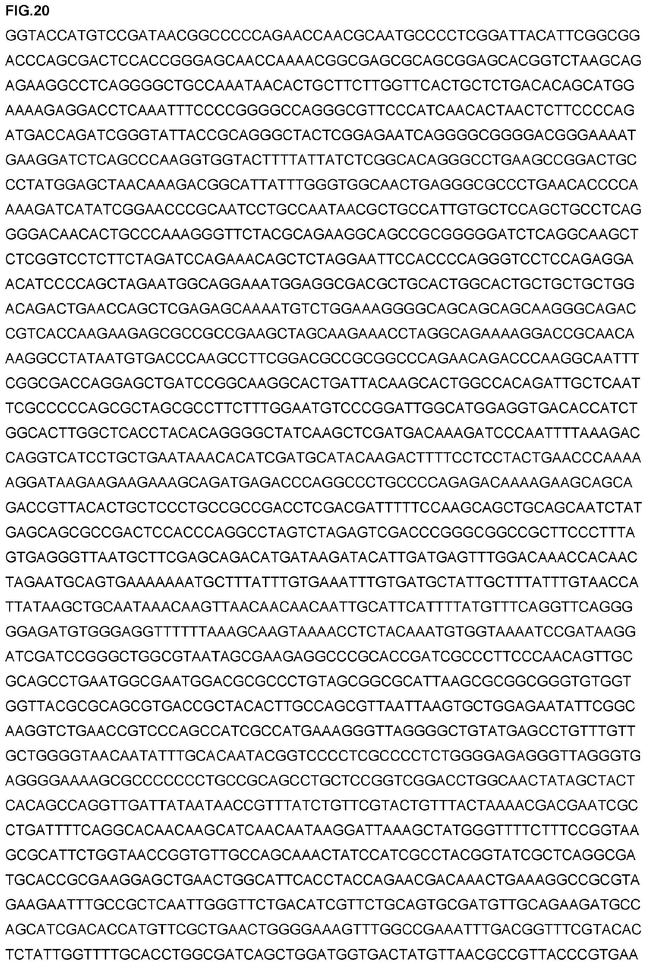

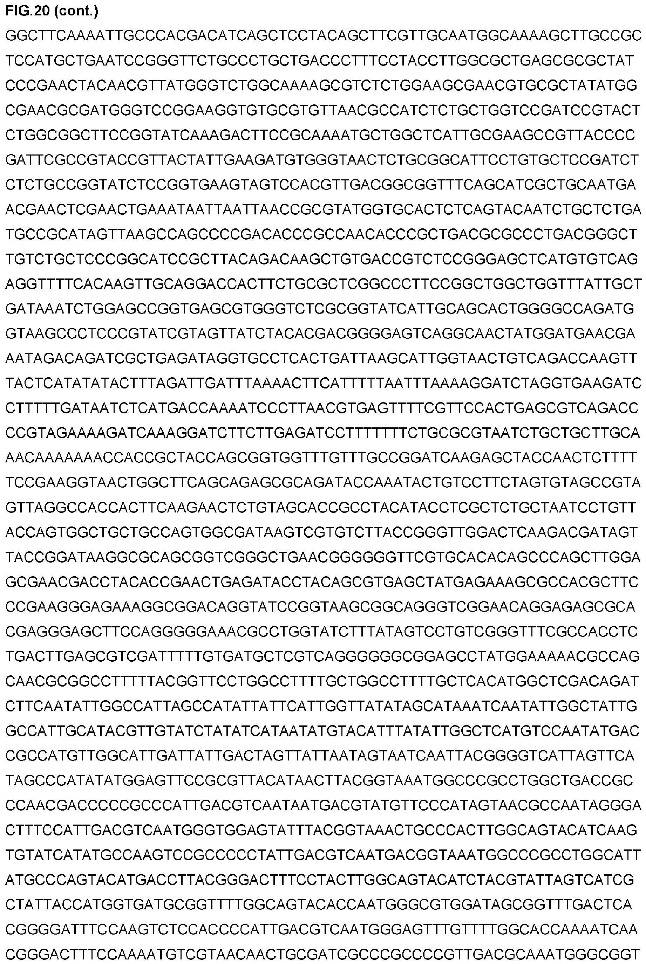

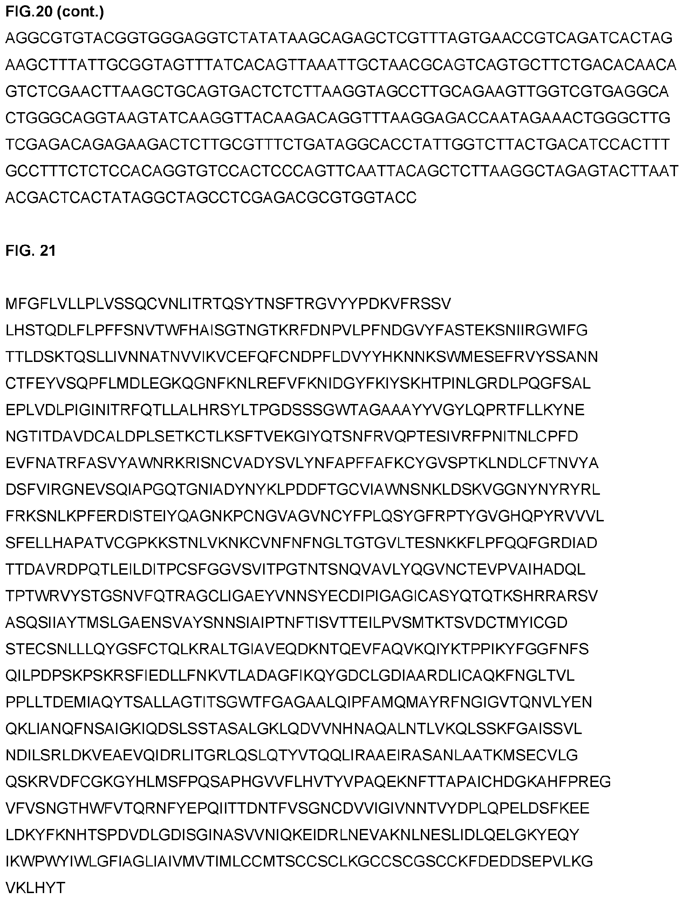

**Figure 20****.** It corresponds to SEQ ID NO: 13; pPAL-N (codon-optimized N gene). **Figure 21****.** It corresponds to SEQ ID NO: 24; S protein Omicron Subvariant BA.4 (GenBank: UUB32912.1).

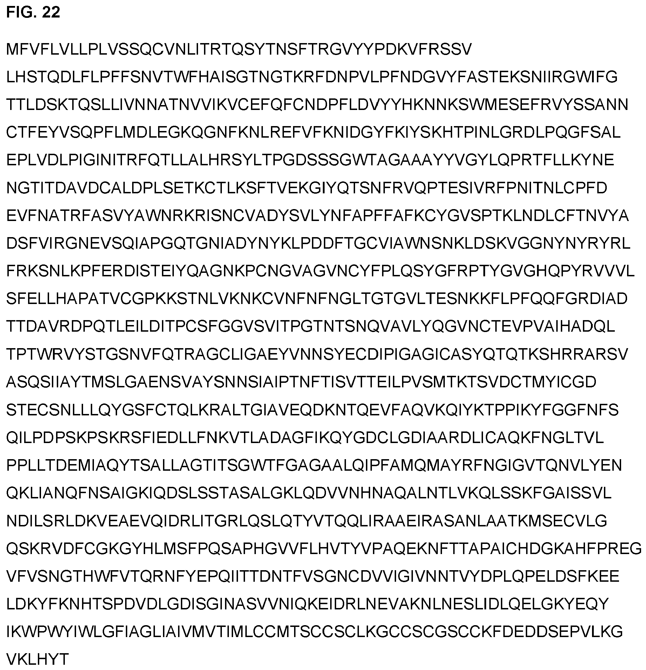

**Figure 22****.** It corresponds to SEQ ID NO: 25; S protein Omicron Subvariant BA.5 (GenBank: UUB32864.1).

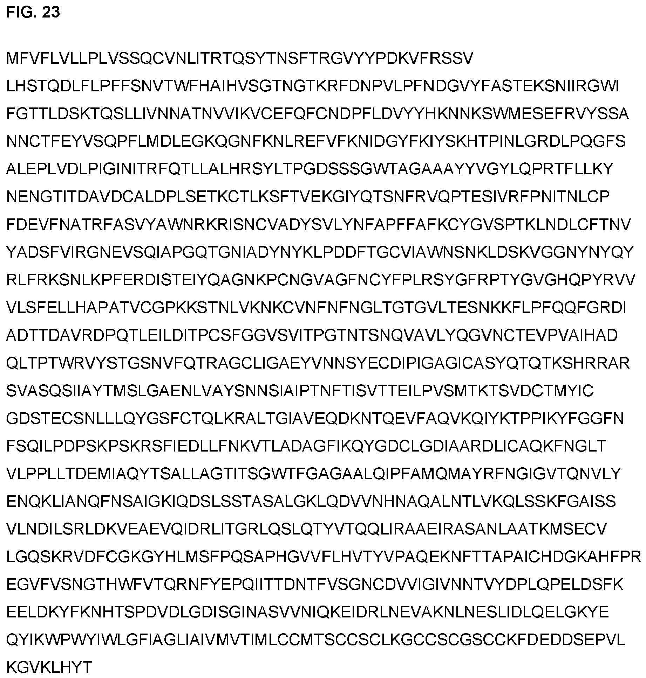

**Figure 23****.** It corresponds to SEQ ID NO: 26; S protein Omicron Subvariant BA2.12.1 (GenBank: UUB32948.1).

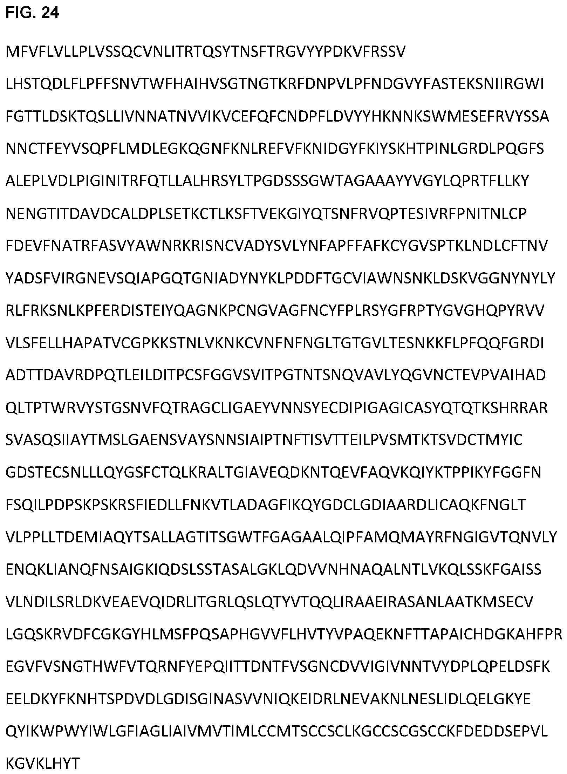

**Figure 24****.** It corresponds to SEQ ID NO: 27; S protein Omicron Subvariant BA2.9.1 (GenBank: UUA48363.1).

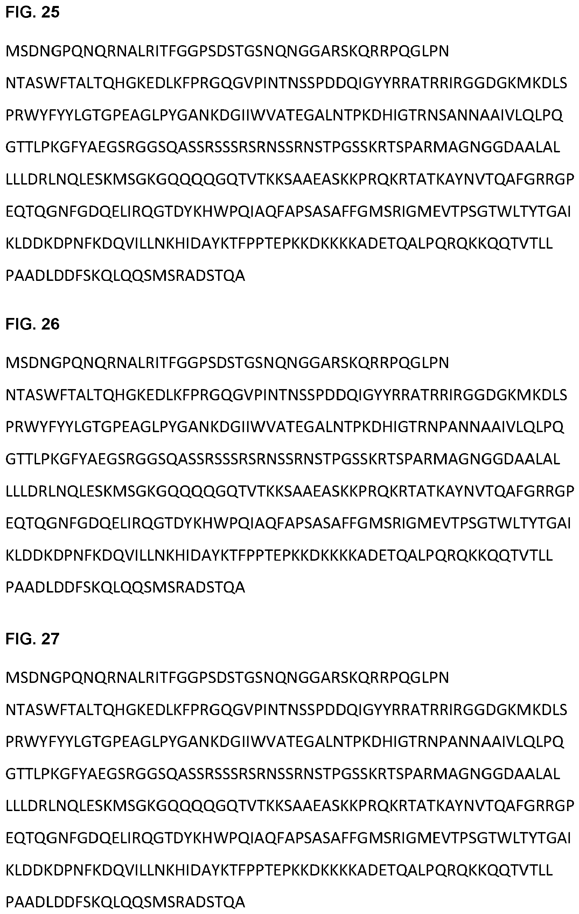

**Figure 25****.** It corresponds to SEQ ID NO: 29; N protein Omicron Subvariant BA.4 (GenBank: UUB32920.1).
**Figure 26****.** It corresponds to SEQ ID NO: 30; N protein Omicron Subvariant BA.5 (GenBank: UUB32872.1).
**Figure 27****.** It corresponds to SEQ ID NO: 31; N protein Omicron Subvariant BA2.12.1 (GenBank: UUB32956.1).

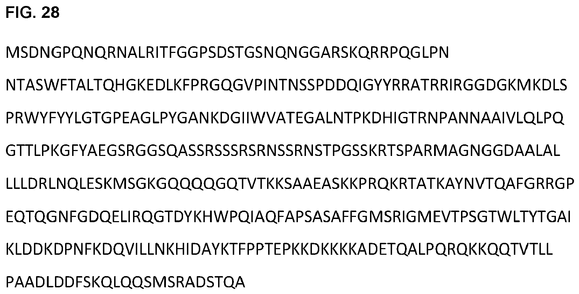

**Figure 28****.** It corresponds to SEQ ID NO: 32; N protein Omicron Subvariant BA2.9.1 (GenBank: UUA48371.1).

### DETAILED DESCRIPTION OF THE INVENTION

### DEFINITIONS

Herein, where the term "combination" is used it is to be understood that this may refer to simultaneous, separate or sequential administration of the components of the combination. In one embodiment "combination" refers to simultaneous administration of the components of the combination. In one embodiment "combination" refers to separate administration of the components of the combination. In one embodiment "combination" refers to sequential administration of the components of the combination.

A "polynucleotide" or "nucleic acid" sequence as used herein refers to a DNA (such as cDNA or genomic DNA) or RNA sequence (such as mRNA, tRNA and rRNA). Both, single-strand as well as double-strand nucleic acid molecules are encompassed by this term. Further included are nucleic acid mimicking molecules known in the art such as synthetic or semi-synthetic derivatives of DNA or RNA and mixed polymers, both sense and antisense strands. Such nucleic acid mimicking molecules or nucleic acid derivatives according to the invention include phosphorothioate nucleic acid. The term captures sequences that include any of the known base analogs of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

A "coding sequence" or a sequence which "encodes" a gene product as used herein, refers to a nucleic acid sequence which is transcribed (in the case of DNA) and translated (in the case of mRNA), in vitro or in vivo when placed under the control of appropriate regulatory sequences.

The term "identity" as used herein refers to an exact nucleotide-to-nucleotide or amino acid to amino acid correspondence of two polynucleotides or polypeptide sequences, respectively. Two or more sequences (polynucleotide or amino acid) can be compared by determining their "percent identity. The "percent identity" of two sequences, whether nucleic acid or amino acid sequences, is the number of exact matches between two aligned sequences divided by the length of the shorter sequence and multiplied by 100. Suitable programs for calculating the percent identity or similarity between sequences are well known in the art, such as the NCBI BLAST program, used for example with default parameters (http://www. ncbi. nlm. gov/cgi-bin/BLAST).

The nucleic acid molecule encoding the N and S sequences (either full-length or a fragment thereof) can be combined, preferably in one or separate expression constructs, with additional elements, such as e.g. control sequences.

The term "immunogenic fragment", as used herein, refers to a fragment of the SARS-2-CoV N nucleocapsid protein or S glycoprotein, that maintains the ability of the full length SARS-2-CoV N nucleocapsid protein or S glycoprotein, respectively, to elicit an immunogenic reaction i.e. its ability to stimulate the immune system of a subject, such as e.g. a human subject, to recognize it as foreign, destroy it, and subsequently enable the immune system to protect the subject against the disease for which the vaccine has been developed.

It is well known in the art that an immunogenic protein may be cleaved to yield fragments which retain or essentially retain the immunogenic activity of the full length protein. Such cleavage may include the removal of a given number of N- and/or C-terminal amino acids, or the removal of a number of internal (non-terminal) amino acids. Said number of amino acids to be removed from the termini and/or internal regions may for example be one, two, three, four, five, six, seven, eight, nine, ten, 15, 20, 25, 30, 40, 50 or more than 50. Preferably, the fragment has a length of at least 6 amino acids, more preferably at least 7 amino acids, such as at least 8 amino acids, at least 9 amino acids, at least 10 amino acids, at least 11 amino acids, at least 12 amino acids, at least 13 amino acids, at least 14 amino acids, and most preferably at least 15 amino acids.

Means and methods for determining whether the immunogenic activity is retained are well known in the art and include e.g. experimental means such as the systematic generation of deletion mutants and their assessment in assays for immunogenic activity. For example, an immunogenic reaction of the fragment to be tested can be analysed by standard and routine techniques, e.g. by administering (e.g. via i.v. injection) the fragment of interest to a mammal such as a mouse and analysing the response of immunogenic biood cells and/or factors (e.g. interleukins) after an appropriate time for an immune reaction to occur. This can for example be done by applying serum from the animals inoculated with the fragment to a peptide array comprising the complete antigen. These and other means and methods for determining whether the immunogenic activity is retained are known to the skilled person and have been described in the art.

Such fragments can be identified using any number of epitope mapping techniques, well known in the art. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., US4,708,871; Geysen et al., Proc. Natl. Acad. Sci. USA 1984, 81 :3998-4002; Geysen et al., Molec. Immunol. 1986, 23:709-715. Similarly, conformational epitopes are readily identified by determining spatial conformation of amino acids such as by, e.g.. X-ray crystallography and 2-dimensional nuclear magnetic resonance. Synthetic antigens are also included within the definition, for example, polyepitopes, flanking epitopes, and other recombinant or synthetically derived antigens. See, e.g., Bergmann et al., Eur. J. Immunol. 1993, 23:2777-2781; Bergmann et al., J. Immunol. 1996, 157:3242- 3249; Suhrbier, A., Immunol, and Cell Biol. 1997, 75:402-408.

In accordance with the present invention, the immunogenic activity of a fragment of the SARS CoV-2, either of N nucleocapsid protein or S glycoprotein, is essentially retained if at least 60% of the immunogenic activity of the full-length protein or glycoprotein, respectively, is retained. Particularly, at least 75% or at least 80% or at least 90% of the immunogenic activity of the full-length N nucleocapsid protein and/or S glycoprotein is retained. In a particular embodiment, the immunogenic fragment retain a 95, 96, 97, 98, 99 or 100% of the immunogenic activity. Also in accordance with the invention are fragments having an increased immunogenic activity compared to the full-length N nucleocapsid protein or S glycoprotein, i.e. more than 100% activity. Preferably, the method to determine whether these percentages of immunogenic activity are retained is by measuring the induction of SARS CoV-2 neutralising antibody titers in serum after immunisation, as shown in the appended examples using mice. A direct comparison of the immunogenic activity of such a fragment can be achieved by carrying out the same experiment using a full-length SARS-CoV-2 N nucleocapsid protein as well as the fragment of interest. Alternatively, standard values for the full-length N protein and S glycoprotein can be used.

It is also envisaged in accordance with the present invention that a mixture of the full-length N nucleocapsid protein with one or more fragment(s) thereof is employed. It is also envisaged in accordance with the present invention that a mixture of the full length S-glycoprotein with one or more fragment(s) thereof is employed.

The terms DNA "control sequences" and "control elements" as used herein, are required to ensure expression of the encoded protein after incorporation thereof in the host cell(s) of the organism to be vaccinated, to thereby synthesize the antigen. Regulatory elements are also required to ensure replication of the nucleic acid molecule in particular in those cases where the newly introduced nucleic acid molecule is present as an extra-chromosomal sequence or where regulation via endogenous regulatory elements is not ensured. Non-limiting examples of regulatory elements are promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences/elements need always be present so long as the selected coding sequence is capable of being replicated, transcribed and translated in an appropriate host cell.

The promoter initiates transcription of the nucleic acid molecule and can be used to control the level of gene expression, while the terminator sequence ends transcription. In a particular embodiment, the promoter should be highly active. Promoters are located near the transcription start sites of genes, on the same strand and upstream on the DNA (towards the 5' region of the sense strand). Promoters can be about 100-1000 base pairs long. An "eukaryotic promoter" includes cis-acting elements such as binding sites for activating protein-1 (AP-1), nuclear factor κB (NF-κB), CArG binding factor A (CBF-A), nuclear factor Y (NF-Y) and others, in addition to the TATA box sequence.

Preferably, the vector is a virus, plasmid, cosmid or bacteriophage. The nucleic acid molecule of the present invention may be inserted into any one of several commercially available vectors. Suitable expression vectors which comprise the described regulatory elements are known in the art such as Okayama-Berg cDNA expression vector pcDV1 (Pharmacia), pRc/CMV, pcDNAI, pcDNA3, pSFV1 (In-Vitrogene). Most preferably, the vector is a virus as described in more detail herein below.

The nucleic acid sequences inserted in the vector can e.g. be synthesized by standard chemical synthesis methods, or isolated from natural sources or produced semi-synthetically, i.e. by combining chemical synthesis and isolation from natural sources. Ligation of the coding sequences to transcriptional regulatory elements and/or to other amino acid encoding sequences can be carried out using established methods, such as restriction digests, ligations and molecular cloning. Vector modification techniques belong to the standard techniques in the field of molecular biology and, thus, belong to the scientific knowledge of the skilled person.

"Operably linked" as used herein refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

The expression "pharmaceutically acceptable carrier, additive or excipient " is intended to include a formulation, or substance used to stabilize, solubilize and otherwise be mixed with active ingredients to be administered to living animals, including humans. This includes any and all solvents, dispersion media, coatings, antibacterial and antifungal agents, isotonic and absorption delaying agents, and the like, compatible with pharmaceutical administration. Except insofar as any conventional media or agent is incompatible with the active compound, such use in the compositions is contemplated.

The term "vaccine" as used herein refers to a biological preparation that induces or enhances the protective immunity of an individual to a particular disease caused by a pathogen. Without wishing to be bound by theory, it is believed that a protective immunity arises from the generation of neutralizing antibodies, or from the activation of cytotoxic cells of the immune system, or both. A vaccine typically contains an agent that resembles a disease-causing microorganism, and is often made from weakened or killed forms of the microbe, its toxins or one of its surface proteins.

The vaccine may be in any formulation for vaccines known in the art, such as for example mucosal vaccines, vaccines for intramuscular injection or vaccines for subcutaneous or intradermal injection as well as vaccines for inhalation, such as e.g. as aerosols. Such vaccine formulations are well known in the art. Accordingly, the vaccine may be in liquid, aerosolic, or solid form and may be, inter alia, in the form of (a) solution(s), (a) spray(s), (a) powder(s) or (a) tablet(s) or paste(s).

The term "DNA vaccine" or "DNA-based immunization" as used herein refer to the injection of a DNA vector (typically a bacterial plasmid) which induces an immune response to the antigen encoded by said DNA vector. Once the DNA construct is administered the host cells take up the foreign DNA, expressing the viral gene and producing the corresponding protein inside the cell. This form of antigen presentation and processing induces both MHC and class I and class II restricted cellular and humoral immune responses (Encke, J. et al., 1999, Intervirology, 42:117-124). DNA vaccines usually consist of a bacterial plasmid operably linked to a strong eukaryotic promoter, the gene of interest which encodes for an antigenic peptide and a polyadenylation/transcriptional termination sequences.

The term "adjuvant" or "vaccine adjuvant" as used herein refers to any substance or combination of substances which non-specifically enhances the immune response to an antigen.

The term "therapeutic treatment" as used herein refers to bringing a body from a pathological state or disease back to its normal, healthy state. For instance, wherein said disease is an infection, after infection or after the clinical manifestation of the disease caused by the infection. It is noted that, this term as used herein is not understood to include the term "prophylactic treatment" as defined herein.

The term "prophylactic treatment" as used herein refers to preventing a pathological state. For instance, wherein said disease is an infection, before the infection. It is noted that this term as used herein is not understood to include the term "therapeutic treatment" as defined above.

The term "mammalian subject" or "mammal" as used herein refers to any of the endothermic vertebrates belonging to class Mammalia.

The term "therapeutically effective amount" as used herein refers to an amount that is effective, upon single or multiple dose administration to a mammalian subject (such as a human patient) in the prophylactic or therapeutic treatment of a given disease.

### POLYNUCLEOTIDES CODING FOR S GLYCOPROTEIN AND N NUCLEOCAPSID

The invention provides combinations, pharmaceutical compositions and kits based on a combination of polynucleotides comprising or consisting of nucleic acid encoding the S glycoprotein and the N nucleocapsid protein.

The S glycoprotein structure was determined by electron cryomicroscopy [3, 4]. Trimers of this protein composed of two subunits (S1 and S2) coat the viral envelope. The S1 subunit contains the receptor-binding domain (RBD). When the RBD interacts with the human angiotensin-converting enzyme (hACE2) receptor, proteases, like furin, cleave a specific sequence separating the S1 from the S2 subunit. This step is essential for the viral infection of the host cell. Initially, the inventors used the original S protein sequence from the Wuhan strain (SEQ ID NO: 1), but it was later modified with the aim of avoiding furin cleavage and maintaining the whole pre-recognition S structure. Protein S variants which have lost furin cleavage site have been described in previous works such as in Bryan A. Johnson et al. 2021 (Nature 591, 293-299) and Bryan A. Johnson et al. (Furin Cleavage Site Is Key to SARS-CoV-2 Pathogenesis. bioRxiv, 2020). This modified protein is generally referred hereinafter "furin-stabilized S protein" (Sfs). Inmunization with Sfs may lead to a more efficacious primary blocking response in a post-vaccination interaction with the virus since the whole protein S is presented to the immune system and neutralizing conformational antibodies against the protein before furin cleavage could be generated. In a preferred embodiment, the SfS protein is of SEQ ID NO: 2 used in the examples provided herein. To the best of our knowledge the inventors of the present invention have for the first time provided a codon optimized for expression in human Sfs nucleotide sequence (SEQ ID NO: 6).

In one embodiment of the invention, the S glycoprotein amino acid sequence is selected from the group consisting of SEQ ID NO: 1, SEQ ID NO: 2, the S glycoprotein amino acid sequence of omicron subvariants (see for instance https://www.who.int/en/activities/tracking-SARS-CoV-2-variants/ and Table 2 below), such as omicron subvariant BA.4 (South Africa, January 2022) of SEQ ID NO: 24, omicron subvariant BA.5 (South Africa, January 2022) of SEQ ID NO: 25, omicron subvariant BA2.12.1 (USA, December 2021) of SEQ ID NO: 26, omicron subvariant BA2.9.1 (multiple countries, February 2022) of SEQ ID NO: 27, omicron subvariant BA2.11 (multiple countries, March 2022), omicron subvariant BA2.13 (multiple countries, February 2022) and omicron subvariant BA2.75 (India, May 2022); a sequence with at least 85%, preferably at least 90%, at least 95%, at least 96%, 97%, 98%, 99% or 100% identity to any thereof, and any immunogenic fragment of any thereof, such as the receptor-binding domain (RBD) of SEQ ID NO: 28, corresponding to residues 331 to 524 of SEQ ID NO: 1:NITNLCPFGEVFNATRFASVYAWNRKRISNCVADYSVLYNSASFSTFKCYGVSPTKLNDLCFT NVYADSFVIRGDEVRQIAPGQTGKIADYNYKLPDDFTGCVIAWNSNNLDSKVGGNYNYLYRLF RKSNLKPFERDISTEIYQAGSTPCNGVEGFNCYFPLQSYGFQPTNGVGYQPYRVVVLSFELLH APATV and the corresponding residues in other protein S variants.

In some embodiments, the S glycoprotein variants described herein above are characterized by comprising the RBD domain (SEQ ID NO: 24) or a sequence with at least 95% identity thereto, preferably at least 96%, 97%, 98%, 99% or 100% identity thereto.

**Table 2 - Omicron subvariants under monitoring* (August 02, 2022)**

| **Pango lineage** | **GISAID clade** | **Nextstrain clade** | **Relationship to circulating VOC lineages** | **Genetic features** | **Earliest documented samples** |
|---|---|---|---|---|---|
| BA.4^{#} | GRA | 22A | BA.1 and BA.2 sister lineage | BA.2-like constellation in the spike protein + S:del69/70, S:L452R, S:F486V, S:Q493R reversion | South Africa, Jan-2022 |
| BA.5# | GRA | 22B | BA.1 and BA.2 sister lineage | BA.2-like constellation in the spike protein + S:del69/70, S:L452R, S:F486V, S:Q493R reversion | South Africa, Jan-2022 |
| BA.2.12.1 | GRA | 22C | BA.2 sublineage | BA.2 + S:L452Q, S:S704F | United States of America, Dec-2021 |
| BA.2.75*** | GRA | 22D | BA.2 sublineage | BA.2 + S:K147E, S:W152R, S:F157L, S:I210V, S:G257S, S:D339H, S:G446S, S:N460K, S:Q493R reversion | India, May-2022 |

| | | | | | |
|---|---|---|---|---|---|
| *these subvariants are tracked under Omicron unless/until sufficient evidence arises that the virus characteristics are substantially different from what is known about the VOC they belong to. If this evidence arises, WHO will decide, in consultation with the TAG-VE, if designation of the emerging variant warrants a separate WHO label. ^{#}these lineages have identical constellation of mutations in the spike and the following differences outside the spike: BA.4: ORF7b:L11F, N:P151S, ORF6:D61L, ORF1a:del141/143; BA.5: M:D3N. ***additional mutation outside the spike protein: ORF1a:S1221L, ORF1a:P1640S, ORF1a:N4060S; ORF1b:G662S; E:T11A | | | | | |

The nucleocapsid protein (N) is not located on the viral surface, because SARS-CoV-2 is an enveloped virus.

In another embodiment of the invention, optionally in combination with any of the embodiments provided above or below, the N glycoprotein amino acid sequence is selected from the group consisting of SEQ ID NO: 3, the N glycoprotein amino acid sequence of omicron subvariants, such as omicron subvariant BA.4 (South Africa, January 2022) of SEQ ID NO: 29, omicron subvariant BA.5 (South Africa, January 2022) of SEQ ID NO: 30, omicron subvariant BA2.12.1 (USA, December 2021) of SEQ ID NO: 31, omicron subvariant BA2.9.1 (multiple countries, February 2022) of SEQ ID NO: 32, omicron subvariant BA2.11 (multiple countries, March 2022), omicron subvariant BA2.13 (multiple countries, February 2022) and omicron subvariant BA2.75 (India, May 2022); and a sequence with at least 85%, preferably at least 90%, at least 95%, at least 96%, 97%, 98%, 99% or 100% identity to any thereof, and any immunogenic fragment of any thereof.

In another embodiment of the invention, optionally in combination with any of the embodiments provided above or below:
a) the nucleic acid sequence encoding a SARS-CoV-2 Spike (S) glycoprotein is selected from the group consisting of SEQ ID NO: 4, SEQ ID NO: 5, SEQ ID NO: 6, the nucleotide sequence encoding the S glycoprotein amino acid sequence of omicron subvariants, such as:
   i. omicron subvariant BA.4 (South Africa, January 2022; BioProject: PRJNA708324, BioSample: SAMN30004798; ISOLATE SARS-CoV-2/human/USA/NJ-PHEL-V22029702/2022 ACCESSION OP090182 (COMPLETE GENOME); nts: 21491..25297),
   ii. omicron subvariant BA.5 (South Africa, January 2022; BioProject: PRJNA708324, BioSample: SAMN30004794, ISOLATE SARS-CoV-2/human/USA/NJ-PHEL-V22029686/2022 ACCESSION OP090178 (COMPLETE GENOME); nts: 21500..25306),
   iii. omicron subvariant BA2.12.1 (USA, December 2021; BioProject: PRJNA708324, BioSample: SAMN30004801, ISOLATE SARS-CoV-2/human/USA/NJ-PHEL-V22029988/2022 ACCESSION OP090185 (COMPLETE GENOME), nts: 21500..25312),
   iv. omicron subvariant BA2.9.1 (multiple countries, February 2022; BioProject: PRJNA720050, BioSample: SAMN29986166; ISOLATE SARS-CoV-2/human/USA/GA-CDC-STM-S6XFKT2Q5/2022 ACCESSION OP077461 (COMPLETE GENOME), nts: 21554..25366),
   v. omicron subvariant BA2.11 (multiple countries, March 2022),
   vi. omicron subvariant BA2.13 (multiple countries, February 2022) and
   vii. omicron subvariant BA2.75 (India, May 2022);
   a sequence with at least 85% identity, preferably at least 90%, at least 95%, at least 96%, 97%, 98%, 99% or 100% identity to any thereof, more preferably of the sequences SEQ ID NO: 4-6, and any portion of any thereof encoding an immunogenic fragment; and
b) the nucleic acid sequence encoding a SARS-CoV-2 nucleocapsid (N) is selected from the group consisting of SEQ ID NO: 7, SEQ ID NO: 8, the nucleotide sequence encoding the N glycoprotein amino acid sequence of omicron subvariants, such as:
   I. omicron subvariant BA.4 (South Africa, January 2022; BioProject: PRJNA708324, BioSample: SAMN30004798, ISOLATE SARS-CoV-2/human/USA/NJ-PHEL-V22029702/2022 ACCESSION OP090182 (COMPLETE GENOME); nts: 28187..29437),
   II. omicron subvariant BA.5 (South Africa, January 2022; BioProject: PRJNA708324, BioSample: SAMN30004794, ISOLATE SARS-CoV-2/human/USA/NJ-PHEL-V22029686/2022 ACCESSION OP090178 (COMPLETE GENOME), nts: 28196..29446),
   III. omicron subvariant BA2.12.1 (USA, December 2021; BioProject: PRJNA708324, BioSample: SAMN30004801, ISOLATE SARS-CoV-2/human/USA/NJ-PHEL-V22029988/2022 ACCESSION OP090185 (COMPLETE GENOME), nts: 28202..29452),
   IV. omicron subvariant BA2.9.1 (multiple countries, February 2022; BioProject: PRJNA720050, BioSample: SAMN29986166; ISOLATE SARS-CoV-2/human/USA/GA-CDC-STM-S6XFKT2Q5/2022 ACCESSION OP077461 (COMPLETE GENOME), nts: 28256...29506),
   V. omicron subvariant BA2.11 (multiple countries, March 2022),
   VI. omicron subvariant BA2.13 (multiple countries, February 2022) and
   VII. omicron subvariant BA2.75 (India, May 2022);
   a sequence with at least 85%, preferably at least 90%, at least 95%, at least 96%, 97%, 98%, 99% or 100% identity to any thereof, preferably to any of the sequences SEQ ID NO: 7-8, and any portion of any thereof encoding an immunogenic fragment.

In another embodiment of the invention, optionally in combination with any of the embodiments provided above or below, the nucleic acid sequence encoding a SARS-CoV-2 Spike glycoprotein is at least 90%, 91%, 92%, 93%, 94%, 95%, 96%, 97%, 98%, 99% or 100% identical to SEQ ID NO: 4, SEQ ID NO: 5 or SEQ ID NO: 6. In another embodiment of the invention, optionally in combination with any of the embodiments provided above or below, the nucleic acid sequence encoding a SARS-CoV-2 Spike glycoprotein consists of SEQ ID NO: 6.

In another embodiment of the invention, optionally in combination with any of the embodiments provided above or below, the nucleic acid sequence encoding a SARS-CoV-2 N nucleocapsid protein is at least 90%, 91%, 92%, 93%, 94%, 95%, 96%, 97%, 98%, 99% or 100% identical to SEQ ID NO: 7 or SEQ ID NO: 8. In another embodiment of the invention, optionally in combination with any of the embodiments provided above or below, the nucleic acid sequence encoding a SARS-CoV-2 N nucleocapsid protein consists of SEQ ID NO: 8.

In another embodiment of the invention, optionally in combination with any of the embodiments provided above or below, the combination, pharmaceutical composition or kit comprises the nucleic acid SEQ ID NO: 6 and SEQ ID NO: 8.

In another embodiment of the invention, optionally in combination with any of the embodiments provided above or below, each one of the polynucleotide sequences (A) and (B) forms part of a separate expression construct. In an alternative embodiment, both polynucleotides (A) and (B) form part of the same expression construct.

In one embodiment of the invention, optionally in combination with any of the embodiments provided above or below, the polynucleotide sequences (A) and (B) are, each one, operably linked to control sequences.

In one embodiment, optionally in combination with any of the embodiments provided above or below, polynucleotides (A) and (B) are operably linked to at least one promoter, particularly to a eukaryotic promoter, i.e., which enables the expression of the inserted coding sequence in eukaryotic cells, for instance, in mammalian cells.

In one embodiment said promoter is a strong constitutive promoter which will ensure high expression levels of the coding sequence, such as for instance viral promoters, including cytomegalovirus immediate early (CMV-IE) promoter, the simian virus 40 (SV40) promoter, the Rous sarcoma virus long terminal repeat (RSV-LTR), Moloney murine leukaemia virus (MoMLV) LTR, and other retroviral LTR promoters; and non-viral promoters such as muscle-specific muscle creatine kinase (MCK), ubiquitin C (UBC) and elongation factor 1a (EF1a) promoters, PGK1 promoter (see, Papadakis et al., Current Gene Therapy 2004, 4, 89-113). Preferably, said promoter is the promoter of the Fabl gene (SEQ ID NO: 16):

GTGCTGGAGA ATATTCGGCA AGGTCTGAAC CGTCCCAGCC ATCGCCATGA
AAGGGTTAGG GGCTGTATGA GCCTGTTTGT TGCTGGGGTA ACAATATTTG
CACAATACGG TCCCCTCGCC CCTCTGGGGA GAGGGTTAGG GTGAGGGGAA
AAGCGCCCCC CCTGCCGCAG CCTGCTCCGG TCGGACCTGG CAACTATAGC
TACTCACAGC CAGGTTGATT ATAATAACCG TTTATCTGTT CGTACTGTTT ACTAAAACGA
CGAATCGCCT GATTTTCAGG CACAACAAGC ATCAACAATA AGGATTAAAG CT.

The underlined sequences are key elements of the promoter: the -35 and the -10 boxes followed by the transcription factor (TF)-binding site.

In one embodiment of the invention, optionally in combination with any of the embodiments provided above or below, polynucleotides forms part (each one or both) of an expression construct. By "expression construct" is meant any genetic element, such as a plasmid, phage, transposon, cosmid, chromosome, virus, virion, etc., which is capable of replication when associated with the proper control elements and which can transfer gene sequences between cells.

Said construct might be a viral vector. A number of viral based systems have been used for gene delivery. Many gene therapy clinical trials rely on retroviruses or adenoviruses to deliver the desired gene. Other viruses used as vectors include adeno-associated viruses, lentiviruses, pox viruses, alphaviruses, and herpes viruses. These viruses differ in how well they transfer genes to the cells they recognize and are able to infect, and whether they alter the cell's DNA permanently or temporarily. A comparison of different viral vectors in use for gene therapy together with an overview of their advantages and disadvantages is provided in http://www.genetherapynet.com/viral-vectors.html. In one embodiment of the invention, optionally in combination with any of the embodiments provided above or below, said vector is a plasmid, particularly a circular or linear bacterial DNA.

In an embodiment, optionally in combination with any of the embodiments provided above or below, the bacterial plasmid comprises the polynucleotide sequences (A) and/or (B) and are operably linked at least to a eukaryotic promoter (preferably, a strong promoter) and to a polyadenylation/transcriptional termination sequences. The bacterial plasmid can be grown in bacteria, such as for example *E. coli* and then isolated and prepared in an appropriate formulation (according to the intended route of administration) before being administered to the host. Following administration, the plasmid is taken up by cells of the host where the encoded peptide(s) are produced.

In some embodiments the plasmid further comprise(s) one or moreof the following elements:
i) a multiple cloning site (MCS) or polylinker, which is an artificial sequence present in virtually all cloning vectors consisting of a conglomerate of specific targets for different restriction enzymes allowing cloning of most of the DNA fragments of interest, an example of MCS is the one in the pClneo plasmid (Promega, GenBank: U47120);
ii) polyadenylation and transcription termination signals. Examples of transcription termination signals include, but are not limited to, polyA, SV40polyA, human growth hormone (HGH)polyA, and bovine growth hormone polyA;
iii) a sequence for plasmid encapsidation in phages, such as the f1 origin; and
iv) a bacterial replication origin, such as the replication origin derived from ColE1.

The plasmid(s) may contain one or more antibiotic resistance genes, such as the neomycin phosphotransferase gene (npt or neo^{R}) or the β-lactamase gene (bla or amp^{R}).

In some embodiments, the plasmid(s) are characterized by the absence of antibiotic resistance genes. In some embodiments, optionally in combination with any of the embodiments provided above or below, the plasmid(s) comprises the Fabl gene, such as in the pPAL plasmid.

*Fabl* gene encodes an enoyl-acyl carrier protein (ACP) reductase which has the following sequence (SEQ ID NO: 17):

MGFLSGKRILVTGVASKLSIAYGIAQAMHREGAELAFTYQNDKLKGRVEEFAAQLGSDIVLQCD
VAEDASIDTMFAELGKVWPKFDGFVHSIGFAPGDQLDGDYVNAVTREGFKIAHDISSYSFVAMA
KACRSMLNPGSALLTLSYLGAERAIPNYNVMGLAKASLEANVRYMANAMGPEGVRVNAISAGP
IRTLAASGIKDFRKMLAHCEAVTPIRRTVTIEDVGNSAAFLCSDLSAGISGEVVHVDGGFSIAAM
NELELK

Enoyl-acyl carrier protein (ACP) reductase is an enzyme which is essential for bacterial fatty acid synthesis and sensitive to triclosan (5-chloro-2-(2,4-dichlorophenoxy)phenol) which was previously known as a selection marker for molecular cloning (see for instance, Goh et al. 2008, BMC Biotechnology, 8:61). The presence of triclosan affects the growth of the bacteria and is lethal beyond a certain concentration in the medium. The selection mechanism in this case is not based on the existence of a gene for resistance to the selection agent, but on increasing the concentration of the enoyl-ACP reductase protein in the cytoplasm in those clones which acquire the foreign DNA. Thus, bacteria acquiring said polynucleotide, recombinant or not, will be able to express the *fab I* gene at much higher levels than non-transformants, for example, as up to about 50 enoyl-ACP reductase molecules per cell can coexist. When the triclosan:enoyl-ACP reductase binding occurs equivalent to equivalent, there will be a range of concentrations of triclosan in which transformant bacteria survive, and non-transformants will not survive. Therefore, this system acts as a selection marker without being necessary the presence of an antibiotic resistance gene.

Gene delivery systems are well known in the art and can be used in the method of the invention. This includes viral and non-viral delivery systems. In preferred embodiments, the vector of the invention is a plasmid and physical and/or chemical methods are being used for gene delivery. Non-viral physical methods of gene delivery include but are not limited to needle injection, pressure injection (e.g., into the mucosa or epidermis), electroporation, ballistic DNA injection, sonoporation, photoporation, magnetofection, and hydroporation. The non-viral chemical methods for gene delivery use synthetic or natural compounds to form particles that facilitate the transfer of genes into the cells. The synthetic vector has an ability to interact with RNA or DNA electrostatically and bind to compact the genetic information in the accommodation of larger genetic transfers. Then, non-viral chemical vectors are able to enter cells by endocytosis. These are typically liposomes and polymers. Liposome based non-viral vectors use liposomes to facilitate gene delivery by the formation of lipoplexes. The lipoplexes form spontaneously when the negatively charged DNA contacts with positively charged liposomes. The polymer-based non-viral vectors use the polymers to interact with DNA to form polyplexes. These can be based for instance in cationic polymers, such as chitosan derivatives, poly(ethylenimine)s, including PEI and derivatives thereof, such as branched or liner poly(Ethylenimine)-cholesterol conjugates, or poly(L-lysine) polymers. In preferred embodiments, gene transfer is conducted by non-viral physical methods of gene delivery.

In one embodiment of the invention, optionally in combination with any of the embodiments provided above or below, the combination, pharmaceutical composition or kit comprises:
a plasmid, as defined in any of the above embodiments, comprising a polynucleotide (A) which is selected from the group consisting of sequence SEQ ID NO: 9, 10, 11, and a sequence with at least 85% of identity to any of them; and
a plasmid, as defined in any of the above embodiments, comprising the polynucleotide (B) which is selected from the group consisting of sequence SEQ ID NO: 12, 13, and a sequence with at least 85% of identity to any of them.

In one embodiment of the invention, optionally in combination with any of the embodiments provided above or below, the combination, pharmaceutical composition or kit comprises:
a plasmid, as defined in any of the above embodiments, comprising a polynucleotide (A), which consists of SEQ ID NO: 11 or a sequence with at least 85% of identity to any of them; and
a plasmid, as defined in any of the above embodiments, comprising the polynucleotide (B), which consists of SEQ ID NO: 13 or a sequence with at least 85% of identity to any of them.

In another embodiment of the invention, optionally in combination with any of the embodiments provided above or below, the combination, pharmaceutical composition or kit comprises a single expression construct, particularly a plasmid, comprising both polynucleotides (A) and (B), as defined in any of the above embodiments.

### THE POLYNUCLEOTIDE OF THE INVENTION FOR USE AS A MEDICAMENT

A second aspect of the invention relates to the combination, pharmaceutical composition or kit for use as a medicament, preferably for use as a vaccine.

It further relates to the polynucleotide sequence as described herein for use in the therapeutic or prophylactic treatment of a disease, for use in enhancing or inducing a prophylactic or therapeutic immune response in a mammalian subject and to a method of therapeutic or prophylactic treatment wherein such treatment comprises administering to a mammalian subject a therapeutically effective amount of said polynucleotide.

Further details and embodiments of said polynucleotides have been provided under the first aspect of the invention.

### VACCINES AND KITS OF THE INVENTION

Also provided herein are vaccines and kits comprising the constructs as described herein.

The kit can comprise one or multiple containers or vials of the constructs described herein, together with instructions for their administration to a subject.

In certain embodiments, optionally in combination with any of the embodiments provided above or below, the subject is a human.

In certain embodiments, optionally in combination with any of the embodiments provided above or below, the instructions indicate that the plasmids are administered to the subject in a single dose, or in multiple (i.e., 2, 3, 4, etc.) doses.

In certain embodiments, optionally in combination with any of the embodiments provided above or below, the instructions indicate that the expression constructs are simultaneously administered to the subject.

In certain embodiments, the instructions indicate that the expression constructs are administered in a single dose to naïve or non-naïve subjects.

In one embodiment, optionally in combination with any of the embodiments provided above or below, the vaccines and kits comprise a single dose to be administrated to a subject in need thereof.

In another embodiment, optionally in combination with any of the embodiments provided above or below, the instructions indicate that the expression constructs are administered in a first (priming) and second (boosting) administration to naive or non-naive subjects.

In one embodiment, optionally in combination with any of the embodiments provided above or below, the kit comprises at least two vials or containers for prime and boost immunization, comprising the plasmids, either separately or in a single formulation, as described herein for a first inoculation ("priming inoculation") in a first vial/container and for an at least second and/or third and/or further inoculation ("boosting inoculation") in a second and/or further vial/container (incorporating for each boosting inoculation the plasmids in separate or in a common container/vial).

In one embodiment, optionally in combination with any of the embodiments provided above or below, the polynucleotides or compositions described herein are administered in a prime-boost regimen which comprises or consists of a first administration and at least a second administration in an interval of time, wherein said interval of time can be for instance a 1, 2, 3, 4, 5, 6, 7, 8, 9, 10 ,11,12, 13, 14, 15, 16, 17, 18, 19, 20 or 21- day interval, preferably a 7, 14 or 21-day interval.

In one embodiment, optionally in combination with any of the embodiments provided above or below, the prime-boost regimen comprises or consists of the administration of the polynucleotides A and B twice with at least a 7-day interval (administration at days 1 and 8), preferably with a 14-day interval (administration at days 1 and 15).

In another embodiment, optionally in combination with any of the embodiments provided above or below, one or more additional booster doses are administered.

In another embodiment, optionally in combination with any of the embodiments provided above or below, the administration of a single prime-boost regimen treats or prevents COVID-19 and/or persistent COVID-19 by providing a substantially complete elimination of SARS-CoV-2 replication as determined by SARS-CoV-2 mRNA detection in the organs of the subject, such as the lungs, heart and/or brain. Routine well-known quantitative technique can be used to determine the replication degree, such as PCR (such as qPCR). Alternatively, a statistically significant reduction with respect to non-vaccinated individuals can also be use.

In another embodiment, optionally in combination with any of the embodiments provided above or below, a single prime-boost administration results in a substantially complete elimination of SARS-CoV-2 replication as determined by viral mRNA detection in the lungs at least by day 7 post-booster (7dpv) administration and/or in the heart and/or brain at least by day 7 post-booster (7 dpv), preferably by day 4 post-booster (4 dpv).

### PHARMACEUTICAL COMPOSITIONS

The present invention also provides pharmaceutical compositions comprising the polynucleotides and expression constructs defined in any of the above embodiments, further comprising and a pharmaceutically acceptable carrier, additive or excipient.

An eight aspect of the invention relates to a pharmaceutical composition comprising a polynucleotide sequence as defined in the second and subsequent aspects of the invention and further comprising a pharmaceutically acceptable carrier, additive or excipient.

Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, that the preparation will contain a pharmaceutically acceptable excipient that serves as a stabilizer, particularly for peptide, protein or other like molecules if they are to be included in the vaccine composition. Examples of suitable carriers that also act as stabilizers for peptides include, without limitation, pharmaceutical grades of dextrose, sucrose, lactose, trehalose, mannitol, sorbitol, inositol, dextran, and the like. Other suitable carriers include, starch, cellulose, sodium or calcium phosphates, citric acid, tartaric acid, glycine, high molecular weight polyethylene glycols (PEGs), and combination thereof.

Certain facilitators of polynucleotide molecules uptake and/or expression ("transfection facilitating agents") can also be included in, e. g, non-viral vector compositions, for example, facilitators such as bupivacaine, cardiotoxin and sucrose, and transfection facilitating vehicles such as liposomal or lipid preparations that are routinely used to deliver nucleic acid molecules. Anionic and neutral liposomes are widely available and well known for delivering nucleic acid molecules.

Cationic lipid preparations are also well-known vehicles for use in the delivery of polynucleotide molecules. Suitable lipid preparations include DOTMA (N-[1-(2,3dioleyloxy) propyl]-N, N, N-trimethylammonium chloride), available under the trade name Lipofectin, and DOTAP (1, 2-bis (oleyloxy)-3 (trimethylammonio) propane). These cationic lipids may preferably be used in association with a neutral lipid, for example DOPE (dioleyl phosphatidylethanolamine).

Alternatively, the polynucleotide sequences of the present invention may be encapsulated, adsorbed to, or associated with, particulate carriers. Examples of particulate carriers include those derived from polymethyl methacrylate polymers, as well as microparticles derived from poly(lactides) and poly(lactide-co-glycolides-), known as PLG. Other particulate systems and polymers can also be used, for example, polymers such as polylysine, polyarginine, polyornithine, spermine, spermidine, as well as conjugates of these molecules.

In a particular embodiment, said pharmaceutical composition is a vaccine, with or without addition of an adjuvant substance or composition.

In a particular embodiment, said pharmaceutical composition comprises at least a further adjuvant, as defined above. Preferred adjuvants include any substance that enhances the immune response of a subject to the antigens encoded by the polynucleotide of the invention. They may enhance the immune response by affecting any number of pathways, for example, by stabilizing the antigen/MHC complex, by causing more antigen/MHC complex to be present on the cell surface, by enhancing maturation of antigen presenting cells (APCs), or by prolonging the life of APCs.

Said adjuvant can be a traditional adjuvant such as killed bacteria, bacterial components, aluminum salts, oil emulsions, polysaccharide particles and biopolymers. Preferably, said adjuvant is a molecular adjuvant. Molecular adjuvants are nucleic acid sequences coding for peptides known to stimulate, modify or modulate a host's immune system. Examples of molecular adjuvants for boosting the immunogenicity of DNA vaccines can be plasmids encoding cytokines (e.g., IL-2, IL-12, IFN-γ, GM-CSF or IL-15) as natural immune stimulators. Chemokine-encoding plasmids have also been evaluated for their ability to enhance DNA vaccines. More recently, costimulatory molecules of the TNF ligand/receptor superfamily and signalling molecules have also been investigated as DNA vaccine adjuvants (Saade et al., Expert Rev Vaccines 2012, 11(2):189-209).

### ADMINISTRATION ROUTE AND DOSING SCHEDULE

The polynucleotides and pharmaceutical composition as defined according to the previous aspects of the invention are formulated to be compatible with its intended route of administration. Methods to accomplish the administration are known to those of ordinary skill in the art. This includes, for example, injections, by parenteral routes such as intravenous, intravascular, intraarterial, subcutaneous, intramuscular, intraperitoneal, intraventricular, intraepidural, or others as well as oral, nasal, ophthalmic, rectal, or topical. Sustained release administration is also specifically contemplated, by such means as depot injections or erodible implants. A preferred route of administration is subcutaneous or intramuscular injection. Another preferred route of administration is intranasal administration.

The dose will be selected according to the administration route, treatment regime and/or administration schedule, having regard to the existing toxicity and effectiveness data.

Vaccination strategies based on prime-boost regimens can be used. These are known to enhance responses against difficult pathogens. These strategies typically involve the priming of the immune responses by using an antigen expressed first by a vector, typically a plasmid DNA, followed by one ore more boosts with administration of the same antigen in a different vector, typically a recombinant virus. This strategy is known as heterologous prime-boost regime. Alternatively, a homologous prime-boost regime can be used wherein the same antigen encoding vector is used for the priming and boosting steps. Preferably, said antigen encoding vector is a plasmid DNA.

Accordingly, a particular administration regime for said polynucleotides or pharmaceutical composition is a homologous prime-boost regime, preferably wherein said polynucleotide is a bacterial plasmid polynucleotide as described herein above, more preferably said homologous prime-boost regime comprises or consists of the administration of the pPAL-S or pPAL-Sfs and the pPAL-N vectors described herein (including the codon-optimized versions thereof).

A suitable unit dose for vaccination is 5-20 µg DNA-encoding polynucleotide/ per kg of body weight, and such dose is preferably administered 1, 2 or 3 times (preferably in a homologous regime) and with an interval of 1-3 weeks. For instance, priming is carried out at day -60 and boost at day -30 or -45 before the infection or day of planned exposure to the infectious agent.

In a particular embodiment, said polynucleotides as defined and according to the previous aspects of the invention are used with other drugs to provide a combination prophylactic or therapeutic treatment. These other drugs may form part of the same composition or be provided as a separate composition for administration at the same time or at different time.

Further details and preferred embodiments of said pharmaceutical composition and its medical uses have been provided under the previous aspects of the invention.

### COMBINATION OF POLYNUCLEOTIDES OF THE INVENTION FOR IMMUNIZATION OF A MAMMAL

In a further aspect the invention relates to an antibody, including a fragment thereof, preferably selected from the list consisting of Fab, Fab', Fab'-SH, Fv, scFv, F(ab')2, Vhh, nanobody and diabody, obtained or obtainable after immunization of a mammal with the combination or pharmaceutical composition of the invention, wherein said antibody or fragment thereof is suitable for the prophylactic or therapeutic treatment of a mammalian subject, preferably for use in passive immunization.

In a related aspect, the invention refers to a method for the obtaining of antibodies comprising the immunization of a mammal, with the combination or pharmaceutical composition of the inventio. Said mammal may be a human or a non-human mammal.

Said method may further comprise the isolation and/or purification of the obtained antibodies. Methods for polypeptide isolation and/or purification are well known in the art. Generally, antibody purification comprises a clarification step and once a clarified solution containing the polypeptide of interest has been obtained, its separation from the other proteins produced is usually attempted using a combination of different chromatography techniques. These techniques separate mixtures of proteins on the basis of their charge, degree of hydrophobicity, or size. Several different chromatography resins are available for each of these techniques, allowing accurate tailoring of the purification scheme to the particular protein involved. Affinity chromatography, which exploits a specific interaction between the protein to be purified and an immobilized capture agent, may also be an option for some polypeptides. Preferably, the antibody purification comprises a step of affinity chromatography, more preferably Protein A chromatography.

The term "antibody" is used in the broadest sense and includes fully assembled antibodies, monoclonal antibodies, polyclonal antibodies, multispecific antibodies (e.g., bispecific antibodies), antigen-binding antibody fragments (e.g., Fab, Fab', F (ab')2, Fv, single chain antibodies, diabodies), camelbodies and recombinant peptides comprising the forgoing as long as they exhibit the desired biological activity.

An "antibody fragment" or "antigen-binding antibody fragment" of an antibody hereby is defined as a fragment of an antibody (e.g., a variable region of an IgG) that is sufficient to confer specific antigen binding to the polypeptide, as long as the antibody retains the desired biological activity. An "antigen- binding region" of an antibody typically is found in one or more hypervariable region(s) of an antibody, i.e., the CDR-1 , -2, and/or -3 regions; however, the variable "framework" regions can also play an important role in antigen binding, such as by providing a scaffold for the CDRs.

Nonlimiting examples of antibody fragments include Fab, Fab', F(ab')2, Fv, domain antibody (dAb), complementarity determining region (CDR) fragments, single-chain antibodies (scFv), single chain antibody fragments, diabodies, triabodies, tetrabodies, minibodies, linear antibodies [Johnson G , Wu TT. (2000) Kabat database and its applications: 30 years after the first variability plot. Nucleic Acids Res. 28:214-218]; chelating recombinant antibodies, tribodies or bibodies, intrabodies, nanobodies, small modular immunopharmaceuticals (SMI Ps), an antigen-binding-domain immunoglobulin fusion protein, a camelized antibody, a VHH containing antibody, or muteins or derivatives thereof; and multispecific antibodies formed from antibody fragments [Chothia C, Lesk AM. (1987)].

It will be understood that particular embodiments described in the Examples are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications are herein incorporated by reference to the same extent as if each individual publication or patent application was specifically and individually indicated to be incorporated by reference.

The practice of the present invention will employ, unless otherwise indicated, conventional methods of virology, microbiology, molecular biology and recombinant DNA techniques within the skill of the art. Such techniques are explained fully in the literature.

The use of the word "a" or "an" may mean "one," but it is also consistent with the meaning of "one or more", "at least one," and "one or more than one."

Throughout this application, the term "about" means the indicated value ± 5% of its value, preferably the indicated value ± 2% of its value, most preferably the term "about" means exactly the indicated value (± 0%).

### EMBODIMENTS

1. A combination comprising:
   - a polynucleotide sequence A comprising or consisting of a nucleic acid sequence encoding a SARS-CoV-2 Spike (S) glycoprotein or an immunogenic fragment thereof; and
   - a polynucleotide sequence B comprising or consisting of a nucleic acid sequence encoding a SARS-CoV-2 nucleocapsid (N) protein or an immunogenic fragment thereof.
2. A pharmaceutical composition comprising a therapeutically effective amount of the combination of embodiment 1 together with one or more pharmaceutically acceptable vehicles, excipients and/or carriers.
3. The pharmaceutical composition of embodiment 2, which is a vaccine.
4. A kit of parts comprising:
   - the polynucleotide sequence A as defined in embodiment 1 in a first unit dosage form,
   - the polynucleotide sequence B as defined in embodiment 1 in a second unit dosage form,
   - container means for containing said first and second dosage forms;
   - and optionally, instructions for use.
5. The combination of embodiment 1, the pharmaceutical composition of any of the embodiments 2-3 or the kit of embodiment 4, wherein the S glycoprotein amino acid sequence is selected from the group consisting of SEQ ID NO: 1, SEQ ID NO: 2, the S glycoprotein amino acid sequence of omicron subvariants, such as omicron subvariant BA.4 (South Africa, January 2022) of SEQ ID NO: 24, omicron subvariant BA.5 (South Africa, January 2022) of SEQ ID NO: 25, omicron subvariant BA2.12.1 (USA, December 2021) of SEQ ID NO: 26 and omicron subvariant BA2.9.1 (multiple countries, February 2022) of SEQ ID NO: 27, a sequence with at least 85% of identity to any of these sequences, and any immunogenic fragment of any thereof, such as the receptor-binding domain (RBD) of SEQ ID NO: 28.
6. The combination, pharmaceutical composition or the kit of any of the preceding embodiments, wherein the N glycoprotein amino acid sequence is selected from the group consisting of SEQ ID NO: 3, the N glycoprotein amino acid sequence of omicron subvariants, such as omicron subvariant BA.4 (South Africa, January 2022) of SEQ ID NO: 29, omicron subvariant BA.5 (South Africa, January 2022) of SEQ ID NO: 30, omicron subvariant BA2.12.1 (USA, December 2021) of SEQ ID NO: 31, omicron subvariant BA2.9.1 (multiple countries, February 2022) of SEQ ID NO: 32, omicron subvariant BA2.11 (multiple countries, March 2022), omicron subvariant BA2.13 (multiple countries, February 2022) and omicron subvariant BA2.75 (India, May 2022); and a sequence with at least 85% of identity to any thereof, and any immunogenic fragment thereof.
7. The combination, pharmaceutical composition or the kit of any of the preceding embodiments, wherein:
   - the nucleic acid sequence encoding a SARS-CoV-2 Spike (S) glycoprotein is selected from the group consisting of SEQ ID NO: 4, SEQ ID NO: 5, SEQ ID NO: 6, the nucleotide sequence encoding the S glycoprotein amino acid sequence of omicron subvariants, a sequence with at least 85% of identity to any of these sequences, and any fragment thereof encoding an immunogenic fragment; and/or
   - the nucleic acid sequence encoding a SARS-CoV-2 nucleocapsid (N) is selected from the group consisting of SEQ ID NO: 7, SEQ ID NO: 8, the nucleotide sequence encoding the N glycoprotein amino acid sequence of omicron subvariants, a sequence with at least 85% of identity to any of these sequences, and any fragment thereof encoding an immunogenic fragment.
8. The combination, pharmaceutical composition or kit of any of the preceding embodiments, wherein the nucleic acid sequence encoding a SARS-CoV-2 Spike (S) glycoprotein is SEQ ID NO: 6 and the nucleic acid sequence encoding a SARS-CoV-2 nucleocapsid (N) is SEQ ID NO: 8.
9. The combination, pharmaceutical composition or the kit of any of the preceding embodiments, wherein each one of the polynucleotide sequences (A) and (B) forms part of a separate expression construct.
10. The combination or pharmaceutical composition of any of the preceding embodiments, wherein both polynucleotides (A) and (B) form part of the same expression construct.
11. The combination, pharmaceutical composition or the kit of any of the preceding embodiments, wherein the polynucleotide sequences (A) and (B) are, independently, operably linked to control sequences.
12. The combination, pharmaceutical composition or the kit of any of the preceding embodiments 9-11, wherein the expression construct is a plasmid, preferably a circular or linear bacterial DNA plasmid.
13. The combination, pharmaceutical composition or the kit of any of the preceding embodiments, comprising:
   - a plasmid comprising a polynucleotide (A) which is selected from the group consisting of sequence SEQ ID NO: 9, 10, 11, and a sequence with at least 85% of identity to any of them; and
   - a plasmid comprising the polynucleotide (B) which is selected from the group consisting of sequence SEQ ID NO: 12, 13, and a sequence with at least 85% of identity to any of them.
14. A combination as defined in any of the preceding embodiments for use as a medicine, particularly as a vaccine.
15. A combination, pharmaceutical composition or a kit as defined in any of the preceding embodiments for use in a method for preventing or treating COVID-19 and/or persistent COVID-19 in a subject, the method comprising the simultaneous or sequential administration of the polynucleotides A and B.
16. The combination, pharmaceutical composition or a kit as defined in any of the preceding embodiments for use as claimed in embodiment 15, wherein polynucleotides A and B are simultaneously administered in separate plasmids.
17. The combination, pharmaceutical composition or a kit as defined in any of the preceding embodiments for use as claimed in any one of the embodiments 15-16, wherein polynucleotides are intramuscuslar administered.
18. The combination, pharmaceutical composition or kit as defined in any of the preceding embodiments for use as claimed in any one of the embodiments 15-17, wherein the polynucleotides are administered in a prime-boost regimen which comprises or consists of the administration of the polynucleotides A and B at least twice within a given interval ot time.
19. The combination, pharmaceutical composition or kit as defined in any of the preceding embodiments for use as defined in any one of the embodiments 15-18, wherein said prime-boost regimen comprises or consists of the administration of the polynucleotides A and B twice with at least a 7-day interval (administration at days 1 and 8), preferably with a 14-day interval (administration at days 1 and 15).
20. The combination, pharmaceutical composition or a kit as defined in any of the preceding embodiments for use as defined in any one of the embodiments 15-19, wherein said method comprises one or more additional booster doses.
21. A method for the obtaining of antibodies comprising the immunization of a mammal other than a human, such as a non-human primate, a cow, a goat, a pig or a horse, with the combination, the pharmaceutical composition or the kit as defined in any of the preceding embodiments 1-13.

### EXAMPLES

### MATERIAL AND METHODS

pPAL constructs. DNA vaccines require a vector to replicate genes and express encoding antigens. Antibiotic resistance genes are often used as selection markers, which must not be released to the environment upon final product commercialization. Then, the use of antibiotic resistance-free vectors is imperative. The pPAL mammalian expression plasmid vector is based on the cytomegalovirus enhancer and promoter sequences. This plasmid does not replicate in mammalian cells and does not contain selectable markers based on antibiotic resistance. The selectable marker is the *E. coli fabl* gene, which encodes for the enoyl-ACP reductase. This enzyme is inhibited by the bacteriostatic compound triclosan, which is the selection agent at an optimal concentration of 3µM [5]. The pPAL-Sfs construct (SEQ ID NO: 11; Figure 1A) contains a modified version of the SARS-CoV-2 spike S glycoprotein-encoding gene with NCBI acc. no. NC045512 (reference genome), gene ID 43740568. First, the sequence was optimized for human expression by the Monte Carlo approach according to relative codon usage frequencies. Second, the cleave site was modified to avoid furin cleavage. Accordingly, the amino acid sequence PRRA (residues 681-684 of SEQ ID NO: 1) was amended toPGGS (residues 681-684 of SEQ ID NO: 2). For this purpose, the nucleotide sequence CCTCGGCGGGCA (SEQ ID NO: 14) was replaced by CCAGGCGGCAGC (SEQ ID NO: 15) (nucleotides 2041-2052 of SEQ ID NO: 11). The pPAL-N construct (SEQ ID NO: 12) contains the SARS-CoV-2 nucleocapsid N protein with NCBI acc. no. NC045512 (reference genome), gene ID 43740575 The Kpnl site-flanked pPAL-Sfs and pPAL-N constructs were obtained by gene synthesis in the pGH vector (ATG Biosynthetics) and transferred to the *E. coli* SURE2 strain by electroporation at 1800 V, 200Ω, and 25 µF. Selection in LB-agar medium was performed with 3 µM triclosan (Sigma). pGH was excised by Kpnl (NEB) digestion and the vaccine constructs circularized with T4 DNA ligase (NEB). Endotoxin-free pPAL, pPAL-Sfs, and pPAL-N plasmid preparations were obtained with PureLink^{™} Expi Endotoxin-Free Giga Plasmid Purification Kit (Invitrogen) following the manufacturer's instructions.

Expression of the antigen genes in HEK293 transfected cells. HEK293 cells (ATCC^{®} CRL-1573^{™}) were grown at 37°C in a 5% CO₂ atmosphere in complete medium containing DMEM supplemented with 10% heat-inactivated fetal bovine serum (Sigma), 100 IU penicillin - 100 µg/mL streptomycin. Semi-confluent HEK293 cells were detached and washed once with 1 mL sterile PBS and once with 0.3 mL GTporator^{®}-M (Protean) per 3 × 10⁶ cells. The cells were transfected by electroporation with pPAL, pPAL-S, pPAL-Sfs, and pPAL-N, and a mock transfection control was also performed. Each transfection of 1.2 × 106 cells with 5 µg of DNA was performed at 220 V, 25Ω, and 950 µF in 80 µL of GTporator^{®}-M solution in a sterile 2 mm-gap cuvette (BTX) using an ECM 630 Electro Cell Manipulator Precision Plus^{®} (BTX). 1.2 mL of pre-warmed complete medium was immediately added to the cells. 0.2 mL of the cell suspension were placed in a 8-well culture slide (Nunc) and 1 mL in a 24-well plate. The cell suspensions were incubated at 37 °C for 24 h in a 5% CO₂ atmosphere.

Transfected HEK293 cells in 24-well plates were washed twice with CM and lysed with 50 µL of a buffer containing 25 mM Tris-HCI pH 7.8, 2 mM EDTA, 2 mM DTT, 1% glicerol, and 1% Triton-X100. The protein extracts were quantified by the Bradford method. Each 20 µg protein extract was treated with 8.3 U/µL of TurboNuclease from Serratia marcescens (Sigma-Aldrich) at room temperature for 10 min and prepared for SDS-PAGE in Laemmli buffer (0.12 M Tris-HCI pH 7.8, 4% SDS, 0.02% bromophenol blue, 5% v/v β-mercaptoethanol, and 20% glicerol), heated at 95°C for 5 min, and run at 30 mA for 90 min in 8-20% TGX precast SDS-PAGE gels (BioRad). Pre-stained PAGE Ruler (Fermentas) was the molecular weight marker. The SDS-PAGE runs were performed in a MiniProtean III cell (BioRad) in running buffer containing 2.4 g/L Tris-base, 11.4 g/L glycine, and 1% w/v SDS. Semi-dry transfer was performed onto 0.45 µm nitrocellulose membranes (BioRad) at 1.3 A and 25V for 10 min (high molecular weight transfer) in a TransBlot Turbo device (BioRad) following the manufacturer's instructions. The membranes were blocked with 5% skimmed milk in PBS-0.05% Tween 20 (PBS-Tween) at room temperature under mild shaking for 1 h and washed three times in PBS-Tween for 15, 5, and 5 min, respectively. The rabbit anti-SARS-CoV-2 S polyclonal antibody #ab272504 specifically recognizing the S2 subunit (Abcam) was used at a 1:750 dilution in blocking solution. The rabbit anti-SARS-CoV-2 N polyclonal antibody, kindly provided by Mercedes Domínguez and Inmaculada Moreno (Instituto de Salud Carlos III), was used at a 1:500 dilution in blocking solution. Incubations with these primary antibodies were performed at room temperature for 90 min. After applying the washing steps, a 1 h incubation was carried out with the secondary HRP-conjugated goat anti-rabbit whole IgG polyclonal antibody (DAKO) at a 1:2,000 dilution in blocking solution. After the washing steps, chemoluminescence was developed with ECL Western Blotting Reagent (Amersham) for 1 min. The images were acquired ChemiDoc MP Image System (BioRad). The colorimetric and the chemoluminescence images were merged using the ImageLab 6.1. software (BioRad).

Indirect immunofluorescence experiments were also performed to visualize Sfs and N gene expression in the HEK293 cells transfected with pPAL, pPAL-Sfs, and pPAL-N. Transfected cells in 8-well slides were washed once with 200 µL of hypotonic solution (3:2 water: DMEM) and twice with 1:1 acetone:methanol. Fixation and permeabilization was performed with 1:1 acetone:methanol at -20°C for 10 min. The preparations were air-dried and the wells were carefully removed from the slides. Three 5 min washes with PBS were applied in a Coplin jar. The preparations were then air-dried and blocked with 20µL of a 5% skimmed milk solution in 0.22 µm-filtered PBS-0.05% Tween solution at 37 °C in a humid chamber for 1h. After removing the excess of blocking solution, the anti-S2 and anti-N primary antibodies mentioned above were used at a 1:50 dilution in blocking buffer. A single 5 min wash step was applied. Then, the cells were incubated with 20 µL of 1:200-diluted Alexa Fluor 488-conjugated goat anti-rabbit IgG secondary antibody (Jackson ImmunoResearch) at room temperature in the dark for 1 h. 10 min before the secondary antibody incubation was completed, 20 µL of 10µg/mL DAPI in PBS were added. The slides were washed four times with PBS during 5 min per wash. The slides were mounted with 50 µL Mowiol 4-88 and leave to dry at 4°C for 16 h in the dark. The fluorescence images were acquired with an AF6000 LAS X Widefield System microscope (Leica).

Immunization with pPAL-Sfs + pPAL-N. 87 eight week-old female wild-type C57BL/6J mice (Charles River) were used for immune response analysis, and 140 eight week-old female B6Cg-Tg(K18-hACE2)2Prlmn/J mice (Jackson) were employed in protection experiments. The animals were generally lodged in groups of five, except for four groups of eight C57BL/6J mice employed in the dose-response experiment, always following the space requirements specified in legislation (EU Directive 2010/63 and Spain regulation RD53/2013, modified by RD1386/2018). The mandatory permits to perform the experiments were approved by the Consejo Superior de Investigaciones Científicas (CSIC) Ethics Committee and the Comunidad Autónoma de Madrid. Experimentation with infected mice was carried out in BSL3+ laboratories (CISA-INIA-CSIC). All animals received food and water ad libitum. Animal welfare measures were applied, considering replacement, reduction and refinement. Anesthesia with isofluorane (3% for induction, 1.5% for maintenance) was administered while vaccine inoculations followed by in vivo electroporation was being applied. 0.2 mg/Kg were then added to the drinking water. Environmental enrichment was implemented. The same anesthesia was administered upon sacrifice by intracardiac puncture. All other measures specified in the regulations were applied. The final point criterium was adopted when appropriate. In the specific case of the hACE2 mice after viral challenge, euthanasia was immediately applied when animal weight decreased 20% or more and when any incipient sign of suffering was detected. The procedures applied in challenge experiments were then subject to retrospective evaluation.

C57BL/6J and K18-hACE2 mice were immunized by the intramuscular route with the pPAL-Sfs + pPAL-N vaccine, which is an endotoxin-free DNA mixture composed of 20 µg pPAL-Sfs and 20 µg pPAL-N in sterile PBS. Immediately after inoculation, *in vivo* electroporation was applied. Two 30G (0.3 × 13 mm) electrode needles connected to an ECM 830 Square Wave Electroporation System (BTX) were placed equidistant to the inoculation point leaving a ~5 mm separation between them and following the direction of the muscular fibers. The negative pole was placed in the posterior position. Six 50 ms 100 V electric pulses were applied in 1 s intervals. The animals were immobilized on an electrically isolated surface and were kept under isofluorane anesthesia (3% induction, 1.5% maintenance) during the procedure. 0.2 mg/mL ibuprofen was administered in drinking water after the procedure. Wild-type C57BL/6J mice were euthanized a week after the booster dose for cellular immune response evaluation in spleen. K18-hACE2 mice were subject to challenge 2 weeks after the booster dose after humoral immune response evaluation.

ELISA. 100 µL of peripheral blood were collected 1 week before vaccination and 15 days post-vaccination. Blood was allowed to clot at 4°C for 5 h. Serum samples were obtained by centrifugation at 8.000xg for 10 min and stored at -20°C. Serial dilutions (1/3) of sera were performed in with 1% BSA in PBS-0.01% Tween 20. 96-well flat-bottom microplates were coated with 50µl of 50 µg/mL RBD (Ray Biotech) in 3.36mM carbonate - 10mM bicarbonate buffer at 4°C for 16 h. After three washing steps with 1% BSA in PBS-0.01% Tween 20, blocking was performed with 3% BSA in PBS-0.01% Tween 20. Next, the washing steps were repeated and 100µL of diluted serum samples were added. Incubations were allowed for 1 h. After washing, incubations with 1:8000-diluted HRP-conjugated protein A (Invitrogen), 1:20,000 goat anti-mouse IgG1, or 1:20,000 goat anti-mouse IgG2c (Bethyl Laboratories) were performed during 1 h. The washing steps were repeated. Color development was performed with TMB Substrate Kit (Thermo Scientific) for 10 min in a volume of 100 µL. The reactions were stopped adding 100µL of 2N H2SO4. A450 was registered with Microplate Reader 680 (BioRad) and Microplate Manager 5.2.1. software (BioRad).

ELIspot and intracellular cytokine staining (ICS). Splenocyte suspensions were obtained in 15 mL of PBS containing 0.3 mM EDTA and 2% HIFBS (wash solution) using 0.45 µm BD Falcon Cell Strainers (BD Biosciences). The cells were harvested by centrifugation at 500xg for 7 min and erythrocites lised during 10 min with 5 mL of a pH 7.2 lysis solution containing 0.15M NH₄Cl, 10 mM KHCO₃, and 0.1 mM EDTA. 25 mL of wash solution was immediately added and the cells were centrifuged at 500xg for 7 min. After an additional wash, the cells were resuspended in 5 mL of RPMI supplemented with 10% HIFBS, 100 UI penicillin - 100 µg/mL streptomycin, and 4.5 µM β-mercaptoethanol. The cell suspensions were filtered through 0.45 µm BD Falcon Cell Strainers (BD Biosciences) and an aliquot was 1:2 diluted with Trypan Blue solution (Sigma) for live cell counting in TC10 Cell Counting Slides using a TC10 Automated Cell Counter (BioRad).

ELIspot assays were performed with Murine IFN-y ELISPOT Kit (Abcam) following the manufacturer's instructions. In the stimulation step, 2 × 10⁵ cells were set in 200 µL of complete proliferation medium containing the appropriate stimulus. Stimulation was performed in triplicate at 37°C for 20 h in a 5% CO₂ atmosphere. The stimuli were: i) 1 µg/mL concanavalin A (Sigma); ii) an equimolar peptide mixture at a final concentration of 25 µg/mL representing the whole S protein sequence prepared from the PepTivator^{®} SARS-CoV-2 Prot_S1 and PepTivator^{®} SARS-CoV-2 Prot_S (Miltenyi) mixtures; iii) an equimolar peptide mixture prepresenting the whole N protein sequence at a final concentration of 25 µg/mL prepared from the PepTivator^{®} SARS-CoV-2 Prot_N (Miltenyi) mixture.

Splenocytes were plated in 96 flat bottom well tissue culture plates (1×10⁶ per well) and stimulated with recombinant protein S (5µg/mL) (SinoBiological). Two hours after protein S addition, cultures were stimulated with peptide S pool (25µg/mL) (Peptivator SARS-CoV-2 Protein S, Miltenyi), with Phorbol 12-Mysristate 13-Acetate (50ng/ml) (Sigma) and lonomycin (1µg/ml) (Sigma) as positive control, or left unstimulated as negative control. Brefeldin-A (5µg/mL), Monensin (2µM) and anti-CD107a antibody (1µg/ml) (PE anti-mouse CD107a; clone 1D4B) (all from Biolegend) were also added to all wells at this stage. Splenocytes were incubated for a further 4 hours in a humidified incubator at 37°C, 5%CO2. Cells were then washed in PBS and stained with viability marker LIVE/DEAD^{™} Fixable Near-IR Dead Cell Stain Kit (Thermofisher) as described in the manufacturer's protocol for 20 min on ice. Cells were the washed in staining buffer (PBS + 2 % FBS + 0.02% sodium azide) and stained for surface cell markers (Alexa Fluor^{®} 488 anti-mouse CD8a; clone 53-6.7; Brilliant Violet 510^{™} anti-mouse CD4; clone GK1.5; PerCP/Cyanine5.5 anti-mouse CD3; clone 17A2; All from Biolegend) for 20 min on ice. Cells were washed twice in staining buffer and fixed and permeabilized using the Cytofix/Cytoperm kit (BD biosciences) according to the manufacturer's instruction for 20 min on ice and stained for intracellular cytokines 30 min on ice (Brilliant Violet 421^{™} anti-mouse TNF-α; clone MP6-XT22; Alexa Fluor^{®} 647 anti-mouse IFN-γ; clone XMG1.2; clone JES6-5H4; PE/Cyanine7 anti-mouse IL-10; clone JES5-16E3; Brilliant Violet 605^{™} anti-mouse IL-4; clone 11B11 , All from Biolegend). After washing as indicated in the Cytofix/Cytoperm kit, cells were resuspended in staining buffer and acquired on a FACSCelestaSORP flow cytometer (BD). Data analysis was performed with FlowJo Software (BD).

SARS-CoV-2 maintenance and infectious challenge. The SARS-CoV-2 MAD6 viral strain was kindly provided by Luis Enjuanes (CNB-CSIC, Madrid, Spain) and was used to perform the infectious challenge. The MAD6 SARS-CoV-2 genome sequence is identical to the Wuhan-Hu-1 isolate (GenBank MN908947). The Calu3 cell line was grown in DMEM medium supplemented with 20% HIFBS and 10 mM HEPES. This cell line was kindly provided by Luis Enjuanes (CNB-CSIC, Madrid, Spain) and was used for viral propagation at a multiplicity of infection (MOI) of 0.001 PFU per cell. The supernatant was harvested 72 h after infection (hpi), subject to three freeze/thaw cycles, and clarified by centrifugation at 1960g for 10 min. The virus was titrated and stored at -80°C until usage. Titration was performed using the Vero E6 cell line (ATCC CRL-1586), which was maintained in complete DMEM medium supplemented with 5% HIFBS. Viral adsorption was allowed for 1 h in 70-80% confluence monolayer Vero E6 cell cultures. Thereafter, 2 mL of 0.5% semisolid agar in complete DMEM medium supplemented with 2% HIFBS were then added. The PFU count was performed after 6 days.

Mice were challenged with 1×10⁵ pfu of MAD6 SARS-CoV2 by the intranasal route 15 days after the booster vaccine dose. Thereafter, the body weight and the clinical profiles were followed daily according to a clinical score sheet (Table 1).

**Table 1. Clinical score evaluation in SARS-CoV-2-infected K18-hACE2 mice.**

| Observation | Score |
|---|---|
| **Body weight** | |
| - No change | 0 |
| - Loss of body weight in % = score points (e.g. loss of 8% body weight = 8 points) | 1-25 |
| - Loss of >25% | 25 |

| **Appearance** | |
|---|---|
| *Fur* | |
| - Shinning | 0 |
| - Matte | 2 |
| - Ruffled | 5 |
| *Eyes* | |
| - Clear | 0 |
| - Unclean, sticky, closed, semi-closed | 5 |
| *Posture* | |
| - Normal | 0 |
| - Hunched | 5 |
| - Massively hunched | 15 |

| **Motility** | |
|---|---|
| - Spontaneous (normal behaviour + social contact) | 0 |
| - Spontaneous but reduced | 1 |
| - Moderately reduced | 5 |
| - Motility only after stimulation | 15 |
| - Coordination disorder | 20 |
| - Lethargy | 25 |

| **Respiration** | |
|---|---|
| - Breathing normal | 0 |
| - Breathing slightly changed | 1 |
| - Accelerated breathing (>30%) | 10 |
| - Strongly accelerated breathing (>50%) | 25 |
| | |

| **Severity rating** | **Sum score** |
|---|---|
| *Severity level 0:* No burden, animals are healthy | 0-5 |
| *Severity level 1:* Low disease burden | 5-10 |
| *Severity level 2:* Moderate disease burden | 10-25 |
| *Severity level 3:* Moderate to severe sickness | 25-50 |
| *Severity level 4:* Severe burden (Implement human endpoint) | >50 |

The clinical score for a given animal is the sum of the registered values. Those animals with a clinical score over 50 were euthanized due to the endpoint criterion established by animal experimentation regulations. Body weight and clinical score follow-up included 10 K18-hACE-mice per group (pPAL control group and pPAL-Sfs + pPAL-N group) and per experiment. 20 mice per group and per experiment were employed for viral burden evaluation in subgroups of 5 at days 2, 4, 7, and 15 after challenge.

### Viral burden evaluation.

Samples of the target organs (lung, heart and brain) obtained on days 2, 4, 7 and 14 post-challenge were lysed applying three freeze-thaw cycles and 20 sonication cycles/min at 5W for 2 min. The lysates were centrifuged at 220g for 5 min to obtain clarified viral stocks. Total RNA extraction was performed with Trizol Reagent (Invitrogen, Waltham, Massachusetts, USA) following the manufacturer's instructions. Two-step qRT-PCR was performed using SuperScript III reverse transcriptase (Invitrogen) and EHF DNA polymerase (Roche) as described by Toussaint et al. [6] using the N1-F (SEQ ID NO: 18: GACCCCAAAATCAGCGAAAT) and N1-R (SEQ ID NO: 19: TCTGGTTACTGCCAGTTGAATCTG) primers, and the N1-P (SEQ ID NO: 20: FAM-ACCCCGCATTACGTTTGGTGGACC-BHQ1). The primers and probe for the β-actin reference gene were ACT_F_1005-1029 (SEQ ID NO: 21: CAGCACAATGAAGATCAAGATCATC), ACT_R_1135-1114 (SEQ ID NO: 22: CGGACTCATCGTACTCCTGCTT), and ACT_P_1081-1105 (SEQ ID NO: 23: JOE-TCGCTGTCCACCTTCCAGCAGATGT-BHQ1). The same clarified viral stocks from target organs were used to titrate viral replication by plaque assays in VERO cells measured as PFU/g tissue.

### RESULTS

### HEK293 cells transfected with the pPAL-Sfs + pPAL-N vaccine candidate expres the SARS-CoV-2 Sfs and N genes

The S and N protein gene sequences were retrieved from the SARS-CoV-2 Wuhan-1 isolate genome sequence (GenBank Acc. No. MN908947). Sfs is a modified version of the S gene that was modified to avoid furin cleavage (2041 CCTCGGCGGGCA → CCAGGCGGCAGC; 681 PRRA → PGGS). The Sfs and the N clones were separately cloned into the pPAL mammalian expression plasmid vector under the control of the CMV enhancer/promoter [5] obtaining the pPAL-Sfs + pPAL-N vaccine candidate (Figure 1 A and B). HEK293 cells transfected with each DNA construct express the respective antigen genes according to Western blot (Figure 1 A and B) and indirect immunofluorescence (Figure 1C) experiments using anti-S2 and anti-N polyclonal antibodies. The S2 fragment is observed in HEK293 cells transfected with pPAL-S but not with pPAL-Sfs.

pPAL-Sfs + pPAL-N vaccination induces SARS-CoV-2-specific humoral and cellular immune responses in mice.

C57BL/6J (B6) mice were immunized with pPAL-Sfs + pPAL-N (20 µg each) by the intramuscular route applying an *in vivo* electroporation procedure following a 15 day-interval prime-boost homologous regime. The average titers of circulating anti-S IgG, anti-RBD IgG, and anti-RBD IgG2c were >2,000. The IgG2c/IgG1 ratio was -750 in pPAL-Sfs + pPAL-N vaccinated mice (Figure 2A). All vaccinated animals were positive to neutralizing antibodies (NAb). The average NAb titers were -100 after the booster dose (Figure 2B). Hence, the pPAL-Sfs + pPAL-N vaccine candidate elicits a SARS-CoV-2-specific neutralizing humoral immune response in mice.

According to an ELISpot assay, all vaccinated mice produced >500 IFN-γ-secreting spot-forming colonies (SFC) per million splenocytes at day 7 post-boost specifically against an 11-mer peptide pool representing the whole SARS-CoV-2 S protein sequence with an overlap of 7 amino acids. The average was -1600 and the maximum was -3100 (Figure 3A). The mean of IFN-γ-secreting SFC per million splenocytes specific to a peptide pool representing the whole SARS-CoV-2 N protein sequence with the same characteristics is much lower (-200) (Figure 3A). 40 µg of pPAL-Sfs produce less IFN-γ-secreting S-specific splenocytes than 20 µg pPAL-Sfs + 20 µg pPAL-N (Figure 3A). A second independent ELIsopt assay confirmed the high levels of IFN-γ-secreting splenocyte clones and showed that pPAL-Sfs + pPAL-N is dose-dependent (>2000, -1500, -1300, and 0 in mice immunized with 20, 10, 5, and 1µg of each plasmid, respectively) (Figure 3B). The T cell response was assessed by intracellular cytokine staining (ICS). The CD107a, IFN-γ, and TNF-α markers were monitored in CD4⁺ and CD8⁺ T cell populations in splenocyte preparations stimulated with the S and N peptide pools. All vaccinated mice produced CD4⁺ and CD8⁺ T cells specifically stimulated against the S and N peptide pools. CD4⁺ and CD8⁺ T cell populations activated against the S peptide pool for the CD107a, IFN-γ, and TNF-α markers were registered (Figure 4A-F). Activation against the N peptide pool was more reduced than against the S peptide pool for all markers, except for CD107a in CD4⁺ T cells. Particularly, no activation against the N peptides was observed for IFN-y (Figure 4A-F). The T cell response is lower with 40 µg of pPAL-Sfs than with 20 µg of pPAL-Sfs + 20 µg of pPAL-N (Figure 4). CD8⁺ T cells showed higher polyfunctionality than CD4⁺ T cells. Patients tested positive for CD8⁺ cells are less sensitive to reinfection [7], which highlights the interest of vaccines eliciting a potent cellular immune response. Approximately 30% of CD8⁺ T cells were positive for three markers (Figure 4G). More precisely, S-specific CD8⁺ T cells expressing CD107a, IFN-y and TNF-α were the more abundant. In summary, vaccination with pPAL-Sfs + pPAL-N triggers robust T cell activation including Th1, cytotoxic CD4⁺, and polyfunctional cytotoxic CD8⁺ T cell populations in vaccinated animals. The finding of cytotoxic CD4⁺ T cells in vaccinated mice is consistent with previous studies. Expression of the N gene potentiates the T cell response against the S protein (Figures 3 and 4).

pPAL-Sfs + pPAL-N vaccination induces full protection in K18-hACE2 transgenic mice

Based on the SARS-CoV-2-specific humoral and cellular immune responses induced by our vaccine candidate, we evaluated the protection efficacy in the K18-hACE2 transgenic mouse model [8]. For this purpose, K18-hACE2 mice were vaccinated with two doses of 20 µg of pPAL-Sfs + 20 µg of pPAL-N with a 15-day time interval, and 2 weeks later were challenged with a lethal dose of SARS-Cov-2 (MAD6 isolate). Before challenge, all vaccinated animals were positive to total anti-RBD IgG, IgG1, and IgG2c, and the titers were high (>2,500) in most animals. The IgG2c titers were higher than IgG1 (Figure 5A). Accordingly, SARS-CoV-2 antibody neutralization titers were also high (Figure 5A), suggesting the induction of a protective immune response. To evaluate protection, clinical signs and body weight after challenge were monitored. Vaccinated mice did not show any weight lost compared with control mice that started to lose weight by day 3 post-challenge (Figure 5B). Furthermore, clinical signs were also significantly reduced in vaccinated mice, whereas the clinical score of non-vaccinated controls increased in clinical score at day 3 post-challenge. All control mice had to be euthanized at 6-7 days post-challenge for ethical reasons (Figure 5C). Hence, complete protection against the disease was achieved in mice vaccinated with two doses of a mixture of plasmids containing Sfs and N encoding genes.

To determine viral replication in vaccinated mice, groups of 5 mice were sacrificed on days 4, 7, and 14 post-challenge. Lungs, hearth, and brain were collected to evaluate viral load by qRT-PCR and to titrate infectious virus by plaque assay. Vaccinated mice showed a significant reduction in SARS-CoV-2 RNA levels in all evaluated organs compared with control mice (Figure 5D). Notably, the majority of vaccinated mice decreased the Ct values by day 4 post-challenge and by Day 14 had no detectable viral RNA in their tissues. The quantification of infectious virus in the different tissues indicated a significant reduction or even undetectable levels in vaccinated mice. Thus, infectious virus was only detected in the lungs of 2 out of 5 mice on day 7 post-challenge and in the heart in 3 out of 5 mice at day 4 post-challenge but with significantly less viral load that control animals (2.2 × 10³ in vaccinates mice versus 9 × 10³ PFUs/gr). Infectious virus in brain showed a sharp difference between vaccinated and control mice. Thus, by day 4 post-challenge either group showed PFUs but by day 7 post-challenge control groups showed a titer of 2.5 × 10³ PFUs/gr tissue in average. All these data indicated that the virus replicated initially in lungs and spread to other organs in control animals, causing death. By contrast, vaccination was able to control the initial replication in lungs and viral spreading, inducing protection.

In order to evaluate whether the pPAL-Sfs + pPAL-N vaccine induced long term protection, K18-hACE2 mice were vaccinated with two doses of 20 µg of pPAL-Sfs + 20 µg of pPAL-N at 15 days interval time, and lethal challenge with SARS-Cov-2 three months after the booster. Antibody titers were still significantly higher in vaccinated mice compared with control mice (Figure 6A) after 3 months, with value of IgG2c higher than IgG1, indicating a Th1 immune response. Protection was determined by weight and clinical signs daily. Control mice lost weight continuously during the 7 days period, and all of them died at or before day 7 post-challenge (Figure 6B). By contrast, pPAL-Sfs + pPAL-N vaccinated mice did not lose weight during the 15 days period. Furthermore, clinical score was significantly higher in the control than in the vaccinated group (Figure 6C). Taken all together, our data indicate that pPAL-Sfs + pPAL-N vaccine induced long term immunity in mice, and accordingly, protection.

pPAL-Sfs and pPAL-N vaccination is effective against the dominant B1.617.2 (Delta) variant.

The continued emergence of variants of concern (VOC) raised concerns that vaccines can provide protective immunity to these new variants. To determine whether the vaccine of the invention based on pPAL-Sfs + pPAL-N was able to induce broad and protective immunity against new variants, we carried out prime-boost vaccination with pPAL-Sfs and pPAL-N in K18-hACE2 mice and challenged with the B1.617.2 (Delta) variant. Thus, K18-hACE2 mice were vaccinated with two doses of 20 µg of pPAL-Sfs + 20 µg of pPAL-N at 15 days interval time, and 2 weeks later were challenged with a lethal dose of SARS-CoV-2 B1.617.2 isolate.

Serum was collected before the challenge and were analyzed by ELISA for the presence of antibodies against the RBD domain of the S protein. Total IgGs, IgG1, and IgG2c were significantly higher than unvaccinated mice (Figure 7A), and IgG2c titers > IgG1a, indicating a Th1 immune response. After challenge with Delta variant, mice were observed daily by clinical signs and body weight. The vaccinated group did not show any weight loss compared with the control group, in which mice died by day 7 post-challenge (Figure 7B). Furthermore, vaccinated mice did not show significant clinical signs compared with the control group (Figure 7C). These data indicate that the pPAL-Sfs + pPAL-N vaccine was able to protect mice against lethal challenge with the Delta variant.

### Discussion

Each one of the plasmids, containing the encoding genes for the Sfs and N proteins of the SARS-CoV-2 Wuhan strain virus, were shown to express properly both proteins in transfected HEK293 cells (Figure 1).

C57BL/6J mice vaccinated with two inoculations of pPAL-Sfs + pPAL-N vaccine delivered by *in vivo* electroporation induced a robust humoral response not only with the production of specific IgGs against the S protein but also against the RBD domain (Figure 2).

Analysis of anti-RBD IgG isotype showed a predominant presence of IgG2c indicating a Th1/Th2 balance skewed towards Th1 (Figure 2). Most of the asymptomatic SARS-CoV-2 infected individuals showed an effective and robust Th1 response. In fact, several published data have shown that the initial establishment of a robust Th1 immune response makes possible to control viral replication, when by contrast, a potent Th2 response correlates with severe forms of the disease [9, 10].

In addition to the generation of specific S protein IgG antibodies, we have demonstrated that immunization with pPAL-Sfs + pPAL-N also induced neutralizing antibodies (Figure 2). Virus neutralization is a measure of antibody efficacy and a correlate of vaccine-induced protection. Thus, the DNA vaccine expressing Sfs and N is able to induce a humoral response compatible with SARS-CoV-2 protection.

T cell responses seem to play a major role in SARS-CoV-2 infection, in which strong-T cell response correlated with recovered patients with mild disease. Actually, a robust CD8⁺-T cell response with broad specificity could be considered a signature of successful protective immunity against SARS-CoV-2. One of the main advantages of DNA vaccines is the ability to stimulate a strong T cell immune response. Our data show the activation of SARS-CoV-2 specific T cell responses to the S and N proteins after pPAL-Sfs + pPAL-N vaccination (Figures 3 and 4). Furthermore, we assessed three functional parameters, CD107a, IFN-γ, and TNF-α, to produce a more in depth understanding of the function and phenotype of the anti-S and anti-N T cells elicited by vaccination.

Cytokine profile analysis of antigen-specific CD4⁺- and CD8⁺-T cells indicated that polyfunctional T CD8⁺ cells were induced by pPAL-Sfs + pPAL-N vaccination to different extent depending on the T cell compartment and the antigen (Figure 4G). Very few activated T CD4⁺ cells expressing all three parameters were detected. We nonetheless found that more than 50% of CD8⁺ T cells specific for the S peptide pool were positive for 2 or more functional parameters, which indicated that these cells are polyfunctional effectors. The T cell response referred to these parameters is lower with 40 µg of pPAL-Sfs than with 20 µg of pPAL-Sfs + 20 µg of pPAL-N (Figure 4). Curiously, anti-N CD8⁺ T cells were less polyfunctional and most exclusively displayed a cytotoxic phenotype. Similarly, both anti-S and anti-N CD4⁺ T cells showed limited polyfunctionality. Nonetheless, both cell populations expressed CD107a in response to peptide stimulation, which indicated that these CD4⁺ T cells possess a cytotoxic phenotype. The presence of cytotoxic CD4⁺ T cells specific for SARS-CoV-2 in murine models has previously been reported, and in some viral infections such as West Nile fever their presence correlates with protection [11]. It would be interesting in future work to determine to which extent these cytotoxic anti-SARS-CoV-2 CD4⁺ T cells contribute to protection. Overall, the data indicate that vaccination induced cellular immune responses to the SARS-CoV-2 antigens included in the vaccine formulation, and that anti-S CD8⁺ T cell effectors are polyfunctional. Since T cell polyfunctionality can be a correlate of protection [12], pPAL-Sfs + pPAL-N vaccination could therefore activate potent T cell effectors capable of recognizing the viral infection.

Our data shows that pPAL-Sfs + pPAL-N can trigger T cell responses to the N antigen. Immunization with the N protein could also trigger antibody responses to the viral nucleoprotein. In some viral infections, non-neutralizing antibodies directed to the nucleoprotein can help clear the infection of enveloped viruses[13-15]. Recently, one of the mechanisms behind the protective role of these anti-N antibodies has been described [16]. It appears that the E3 ubiquitin ligase TRIM21 is able to use anti-N antibodies to target the N protein for proteasomal degradation, which in turn triggers the activation of effective cytotoxic T cell responses against the N antigen [16]. Thus, the inclusion of SARS-CoV-2 N protein in vaccine formulation could be important to improve protection, as it could provide T cell reactivity against this more conserved antigen through multiple mechanisms.

The pPAL-Sfs + pPAL-N vaccine was also capable of protecting mice against the Wuhan strain as well as the VOC B1.617.2 (Delta) (Figures 5 and 7). Therefore, the humoral and cellular immune response triggered against the S and N antigens of SARS-CoV-2 by pPAL-Sfs + pPAL-N was likely capable of protecting mice from the disease. Our analysis of viral load by RT-qPCR and PFU titration in lung, heart and brain tissue homogenates indicated that pPAL-Sfs + pPAL-N vaccination prevented virus spreading to organs that are linked to the serious long-term side effects of COVID-19, such as the heart [7] or the brain (Figure 5D). It is noteworthy that protection was achieved even in animals with low specific antibody titers. This suggests that pPAL-Sfs + pPAL-N could produce humoral and cellular immune responses that synergize and increase the protection afforded by the vaccine. Additionally, protection was also achieved when SARS-CoV-2 challenge was performed 3 months after the last vaccination (Figure 6). Therefore, it appears that pPAL-Sfs + pPAL-N vaccination could induce durable immunity to the disease. The protection data shows that the pPAL-Sfs + pPAL-N vaccine has the potential to protect animals from the disease produced by the original strain of SARS-CoV-2 as well as that of VOC. The pPAL-Sfs + pPAL-N vaccine deserves complementary studies to move for clinical trials with humans.

### References

1. Mallapaty, S., India's DNA COVID vaccine is a world first - more are coming. Nature, 2021. 597(7875): p. 161-162.
2. Smith, C.C., et al., Landscape and Selection of Vaccine Epitopes in SARS-CoV-2. bioRxiv, 2020.
3. Lan, J., et al., Structure of the SARS-CoV-2 spike receptor-binding domain bound to the ACE2 receptor. Nature, 2020. 581(7807): p. 215-220.
4. Wrapp, D., et al., Cryo-EM structure of the 2019-nCoV spike in the prefusion conformation. Science, 2020. 367(6483): p. 1260-1263.
5. Alcolea, P.J., A. Alonso, and V. Larraga, The antibiotic resistance-free mammalian expression plasmid vector pPAL for development of third generation vaccines. Plasmid, 2019. 101: p. 35-42.
6. Toussaint, J.F., et al., Bluetongue virus detection by two real-time RT-qPCRs targeting two different genomic segments. J Virol Methods, 2007. 140(1-2): p. 115-23.
7. Collier, A.Y., et al., Characterization of immune responses in fully vaccinated individuals after breakthrough infection with the SARS-CoV-2 delta variant. Sci Transl Med, 2022. 14(641): p. eabn6150.
8. Winkler, E.S., et al., SARS-CoV-2 infection of human ACE2-transgenic mice causes severe lung inflammation and impaired function. Nat Immunol, 2020. 21(11): p. 1327-1335.
9. Gutierrez-Bautista, J.F., et al., Negative Clinical Evolution in COVID-19 Patients Is Frequently Accompanied With an Increased Proportion of Undifferentiated Th Cells and a Strong Underrepresentation of the Th1 Subset. Front Immunol, 2020. 11: p. 596553.
10. Huang, C., et al., Clinical features of patients infected with 2019 novel coronavirus in Wuhan, China. Lancet, 2020. 395(10223): p. 497-506.
11. Brien, J.D., J.L. Uhrlaub, and J. Nikolich-Zugich, West Nile virus-specific CD4 T cells exhibit direct antiviral cytokine secretion and cytotoxicity and are sufficient for antiviral protection. J Immunol, 2008. 181(12): p. 8568-75.
12. Boyd, A., et al., Correction: Pathogen-Specific T Cell Polyfunctionality Is a Correlate of T Cell Efficacy and Immune Protection. PLoS One, 2015. 10(9): p. e0138395.
13. Mayr, L.M., B. Su, and C. Moog, Non-Neutralizing Antibodies Directed against HIV and Their Functions. Front Immunol, 2017. 8: p. 1590.
14. Bootz, A., et al., Protective capacity of neutralizing and non-neutralizing antibodies against glycoprotein B of cytomegalovirus. PLoS Pathog, 2017. 13(8): p. e1006601.
15. Carragher, D.M., et al., A novel role for non-neutralizing antibodies against nucleoprotein in facilitating resistance to influenza virus. J Immunol, 2008. 181(6): p. 4168-76.
16. Gussow, A.B., et al., Genomic determinants of pathogenicity in SARS-CoV-2 and other human coronaviruses. Proc Natl Acad Sci USA, 2020. 117(26): p. 15193-15199.

## Claims

1. A combination comprising:
- a polynucleotide sequence A comprising or consisting of a nucleic acid sequence encoding a SARS-CoV-2 Spike (S) glycoprotein or an immunogenic fragment thereof; and
- a polynucleotide sequence B comprising or consisting of a nucleic acid sequence encoding a SARS-CoV-2 nucleocapsid (N) protein or an immunogenic fragment thereof.

2. A pharmaceutical composition comprising a therapeutically effective amount of the combination of claim 1 together with one or more pharmaceutically acceptable vehicles, excipients and/or carriers.

3. The pharmaceutical composition of claim 2, which is a vaccine.

4. A kit of parts comprising:
- the polynucleotide sequence A as defined in claim 1 in a first unit dosage form,
- the polynucleotide sequence B as defined in claim 1 in a second unit dosage form,
- container means for containing said first and second dosage forms;
- and optionally, instructions for use.

5. The combination of claim 1, the pharmaceutical composition of any of the claims 2-3 or the kit of claim 4, wherein:
- the S glycoprotein amino acid sequence is selected from the group consisting of SEQ ID NO: 1, SEQ ID NO: 2, the S glycoprotein amino acid sequence of omicron subvariants, such as omicron subvariant BA.4 (South Africa, January 2022) of SEQ ID NO: 24, omicron subvariant BA.5 (South Africa, January 2022) of SEQ ID NO: 25, omicron subvariant BA2.12.1 (USA, December 2021) of SEQ ID NO: 26 and omicron subvariant BA2.9.1 (multiple countries, February 2022) of SEQ ID NO: 27, a sequence with at least 85% of identity to any of these sequences, and any immunogenic fragment of any thereof, such as the receptor-binding domain (RBD) of SEQ ID NO: 28; and/or.
- the N glycoprotein amino acid sequence of omicron subvariants, such as omicron subvariant BA.4 (South Africa, January 2022) of SEQ ID NO: 29, omicron subvariant BA.5 (South Africa, January 2022) of SEQ ID NO: 30, omicron subvariant BA2.12.1 (USA, December 2021) of SEQ ID NO: 31, omicron subvariant BA2.9.1 (multiple countries, February 2022) of SEQ ID NO: 32, omicron subvariant BA2.11 (multiple countries, March 2022), omicron subvariant BA2.13 (multiple countries, February 2022) and omicron subvariant BA2.75 (India, May 2022); and a sequence with at least 85% of identity to any thereof, and any immunogenic fragment thereof.

6. The combination, pharmaceutical composition or the kit of any of the preceding claims, wherein:
- the nucleic acid sequence encoding a SARS-CoV-2 Spike (S) glycoprotein is selected from the group consisting of SEQ ID NO: 4, SEQ ID NO: 5, SEQ ID NO: 6, the nucleotide sequence encoding the S glycoprotein amino acid sequence of omicron subvariants, a sequence with at least 85% of identity to any of these sequences, and any fragment thereof encoding an immunogenic fragment; and/or
- the nucleic acid sequence encoding a SARS-CoV-2 nucleocapsid (N) is selected from the group consisting of SEQ ID NO: 7, SEQ ID NO: 8, the nucleotide sequence encoding the N glycoprotein amino acid sequence of omicron subvariants, a sequence with at least 85% of identity to any of these sequences, and any fragment thereof encoding an immunogenic fragment;
preferably, wherein the nucleic acid sequence encoding the SARS-CoV-2 Spike (S) glycoprotein is SEQ ID NO: 6 and the nucleic acid sequence encoding a SARS-CoV-2 nucleocapsid (N) is SEQ ID NO: 8.

7. The combination, pharmaceutical composition or the kit of any of the preceding claims, wherein each one of the polynucleotide sequences (A) and (B) forms part of a separate expression construct, preferably wherein said expression construct is a plasmid, more preferably a circular or linear bacterial DNA plasmid.

8. The combination or pharmaceutical composition of any of the preceding claims, wherein both polynucleotides (A) and (B) form part of the same expression construct, preferably wherein said expression construct is a plasmid, more preferably a circular or linear bacterial DNA plasmid.

9. The combination, pharmaceutical composition or the kit of any of the preceding claims, comprising:
- a plasmid comprising a polynucleotide (A) which is selected from the group consisting of sequence SEQ ID NO: 9, 10, 11, and a sequence with at least 85% of identity to any of them; and/or
- a plasmid comprising the polynucleotide (B) which is selected from the group consisting of sequence SEQ ID NO: 12, 13, and a sequence with at least 85% of identity to any of them.

10. A combination as defined in any of the preceding claims for use as a medicine, particularly as a vaccine.

11. A combination, pharmaceutical composition or a kit as defined in any of the preceding claims for use in a method for preventing or treating COVID-19 and/or persistent COVID-19 in a subject, the method comprising the simultaneous or sequential administration of the polynucleotides A and B.

12. The combination, pharmaceutical composition or a kit as defined in any of the preceding claims for use as claimed in claim 11, wherein polynucleotides A and B are simultaneously administered, preferably intramuscularly, in separate plasmids.

13. The combination, pharmaceutical composition or kit as defined in any of the preceding claims for use as claimed in any one of the claims 11-12, wherein the polynucleotides are administered in a prime-boost regimen which comprises or consists of the administration of the polynucleotides A and B at least twice within a given interval ot time, optionally wherein said method comprises one or more additional booster doses.

14. The combination, pharmaceutical composition or kit as defined in any of the preceding claims for use as claimed in any one of the claims 11-13, wherein said prime-boost regimen comprises or consists of the administration of the polynucleotides A and B twice with at least a 7-day interval (administration at days 1 and 8), preferably with a 14-day interval (administration at days 1 and 15).

15. A method for the obtaining of antibodies comprising the immunization of a mammal other than a human, such as a non-human primate, a cow, a goat, a pig or a horse, with the combination, the pharmaceutical composition or the kit as defined in any of the preceding claims.
